# EUROPEAN PATENT APPLICATION

(11) **EP 4 421 042 A2**
(43) Date of publication of application: **28.08.2024**
(21) Application number: 24179879.2
(22) Date of filing: 01.12.2017
(51) Int. Cl.: C02F 1/46

(54) **FLUID TREATMENT SYSTEMS AND METHODS OF USING THE SAME**

(30) Priority: 02.12.2016 US 201662429702 P; 09.06.2017 US 201762517340 P; 11.09.2017 US 201762556657 P
(62) Divisional of application: 17875787.8
(71) Applicant: Ethonus, Inc., Tacoma, Washington 98422 (US)
(72) Inventor: FLECKNER, Karen, Tacoma, Washington, 98422 (US); NEYLON, Michael, Tacoma, Washington, 98422 (US)
(74) Representative: Herrero & Asociados, S.L.

(57) **Abstract**

A fluid treatment system that includes a sonic energy generator and an electromagnetic field generator is described herein. The fluid treatment system may include a controller that independently controls the sonic energy generator and the EMF generator while in use. Also described herein are methods of treating a fluid including applying a sonic signal to at least a portion of the fluid, and applying an electromagnetic field signal to at least the portion of the fluid by a direct conductive path. Methods of treating water that has been extracted by an atmospheric water generator unit using such a fluid treatment system are also described herein.

## Description

### BACKGROUND

### Technical Field

Embodiments of the disclosure are generally directed to fluid treatment systems that use sonication and conductive electromagnetic fields, as well as methods of using the same.

### Description of the Related Art

A variety of fluid treatment mechanisms are known in the art, including chemical treatments (e.g., advanced oxidation processes (AOP)), sonication, filtration, and application of electromagnetic fields. Fluid treatment systems typically include one or more treatment mechanisms that are applied to the same volume of fluid in serial. In other words, a first treatment will be used on a volume of fluid in a first location and then the fluid travels to a second location, where the volume of fluid is treated with a second treatment.

However, there are few fluid treatment mechanisms that do not, require the use of chemicals, which may be disadvantageous (e.g., in the case of drinking water), that may be used to treat a variety of fluids having a variety of contaminants with minimal adjustment, and/or that are space and energy efficient given the volume of fluid that may be treated. Accordingly, there remains a need in the art for fluid treatment systems, and related methods, that do not require the use of chemicals, may be used to treat a variety of fluids containing a variety of contaminants, are compact, and are energy efficient. This disclosure provides this and related advantages.

### BRIEF SUMMARY

Aspects of the present disclosure include a fluid treatment system comprising: a sonic energy generator, such as a sonicator that, in use, generates and applies a sonic signal to at least a portion of a fluid in a vessel; and an electromagnetic field (EMF) generator that, in use, conductively applies an EMF signal to at least the portion of the fluid. In embodiments, the fluid treatment system further comprises a first controller that, in use, independently controls the sonic energy generator and the EMF generator. In some embodiments, the fluid treatment system further comprises a sensor that, in use, monitors a condition of the fluid treatment system and transmits feedback regarding the condition to the first controller.

Further aspects of the disclosure include a method comprising: treating a fluid in a vessel, the treating comprising: applying a sonic signal to at least a portion of the fluid; and applying an EMF signal to at least the portion of the fluid by a direct conductive, path. In embodiments, treating the fluid comprises independently controlling, by a first controller, the sonic signal and the EMF signal. In embodiments, the fluid comprises suspended solids, dissolved solids, dissolved gasses, metals, metal salts, inorganic compounds, organic compounds, such as volatile organic compounds, biological materials, radiological materials, algae, bacteria, viruses, or a combination thereof.

In some embodiments, applying the sonic signal cavitates at least a portion of the fluid. In some embodiments, applying the sonic signal, applying the EMF signal, or both causes nucleation. In some embodiments, applying the sonic signal, applying the EMF signal, or both causes sonofragmentation.

Aspects of the present disclosure further include a method, comprising: activating a plurality of atmospheric water generator (AWG) units comprising a first AWG unit and a second AWG unit; extracting water from ambient air by the plurality of AWG units; and treating, at least a portion of the water, the treating comprising: applying a sonic signal to at least the portion of the water; and applying an EMF signal to at least the portion of the water by a direct conductive path. In embodiments, the method further comprises deactivating the second AWG unit based at least on data regarding geography, climate, weather, water, power, or a combination thereof. In some embodiments, the activating the plurality of AWG units is based at least on data regarding geography, climate, weather, water, power, or a combination thereof.

In various embodiments, the first AWG unit, the second AWG unit, or both, have a first setting and a second setting, the first setting having a higher extraction efficiency and a higher energy consumption than the second setting, and the second setting having a lower extraction efficiency and a lower energy consumption than the first setting. In some embodiments, the first AWG unit and the second AWG unit are changed from the first setting to the second setting based at least on data regarding geography, climate, weather, water, power, or a combination thereof.

Additional aspects of the disclosure include a system, comprising: a plurality of AWG units comprising: a first AWG unit; and a second AWG unit; and a water treatment device comprising: a sonic energy generator that, in use, applies a sonic signal to at least a portion of a fluid in a vessel; and an EMF generator that, in use, conductively applies an EMF signal to at least the portion of the fluid. In embodiments, the system further comprises a structure configured to increase mixing of ambient air near the first AWG unit. In some embodiments, the system further comprises a controller that, in use, independently controls the plurality of AWG units, the water treatment device, or both.

### BRIEF DESCRIPTION OF THE SEVERAL VIEWS OF THE DRAWINGS

The detailed description is provided with reference to the accompanying figures. In the figures, the left-most digit(s) of a reference number identifies the figure in which the reference number appears. The use of the same reference numbers in different figures indicates similar or identical components or features.
**FIG. 1** illustrates an embodiment of a fluid treatment system of the disclosure.
**FIG. 2** demonstrates an example of an overlaid sonic signal and EMF signal, where the EMF signal is pulsed at random intervals, having no set correlation to the ultrasound signal according to an embodiment of the disclosure.
**FIG. 3** demonstrates an example of an overlaid sonic signal and EMF signal, where there EMF signal is pulsed at the same frequency, without a phase-shift, as the sonic signal according to an embodiment of the disclosure.
**FIG. 4** demonstrates an example of an overlaid sonic signal and EMF signal, where the EMF signal is pulsed at half the frequency of the sonic signal, without any phase-shift, according to an embodiment of the disclosure.
**FIG. 5** demonstrates an example of an overlaid sonic signal and EMF signal, where the EMF signal is pulsed at twice the frequency of the sonic signal, without any phase-shift, according to an embodiment of the disclosure.
**FIG. 6** demonstrates an example of an overlaid sonic signal and EMF signal, where the EMF signal is pulsed at the same frequency at the sonic signal, with a 90-degree leading phase-shift, according to an embodiment of the disclosure.
**FIG. 7** shows an embodiment of a fluid treatment system of the disclosure.
**FIG. 8** illustrates the effect of relative humidity on the water production rates (gal/day) for two types of AWG units.
**FIG. 9** illustrates the effect of relative humidity on the electrical efficiencies rates (electricity used/gal) for two types of AWG units.
**FIG. 10** shows the average relative humidity as a function of hour of the day for a central Texas location over three days.
**FIG. 11** shows the electrical efficiency of a modeled large scale AWG platform with a 1:2 ratio of large to small AWG units as a function of the relative humidity trigger value for taming on the smaller AWG units.
**FIG. 12** shows the modeled results of changes in surface relative humidity on an area surrounding a large scale AWG platform operating 100 AWG units.
**FIG. 13** shows the modeled results of changes in surface relative humidity on an area surrounding a large scale AWG platform operating 500 AWG units.
**FIG. 14** shows the modeled results of changes in surface relative humidity on an area surrounding a large scale AWG platform operating 1,000 AWG units.
**FIG. 15** shows the modeled results of changes in surface relative humidity on an area surrounding a large scale AWG platform operating 5,000 AWG units.
**FIG. 16** shows the modeled results of changes in surface relative humidity on an area surrounding a large scale AWG platform operating 10,000 AWG units.
**FIG. 17** shows the modeled results of changes in surface temperature on an area surrounding a large scale AWG platform operating 100 AWG units.
**FIG. 18** shows the modeled results of changes in surface temperature on an area surrounding a large scale AWG platform operating 500 AWG units.
**FIG. 19** shows the modeled results of changes in surface temperature on an area surrounding a large scale AWG platform operating 1,000 AWG units.
**FIG. 20** shows the modeled results of changes in surface temperature on an area surrounding a large scale AWG platform operating 5,000 AWG units.
**FIG. 21** shows the modeled results of changes in surface temperature on an area surrounding a large scale AWG platform operating 10,000 AWG units.

### DETAILED DESCRIPTION

Described herein are systems for treating a fluid using sonication and electromagnetic fields, as well as methods for using the same. Such systems include a sonic energy generator, such as a sonicator that generates and applies a sonic signal and an EMF generator that applies a conductive EMF signal to a fluid. The sonic signal and EMF signal may be independently controlled. The systems disclosed herein may be in the form of a standalone device or may be a portion of a larger treatment system.

Both sonic signals and EMF signals individually may result in nucleation (e.g., homogenous nucleation) or crystallization of dissolved species, acoustic cavitation (which causes damage to cell walls or cell membranes of various microorganisms), or both. In embodiments, the sonic signal and conductive EMF signal are applied to the same volume of fluid. In some embodiments, the sonic signal and the conductive EMF signal are applied simultaneously or substantially simultaneously. The simultaneous or substantially simultaneous application of a sonic signal and conductive EMF to a volume of fluid may produce synergistic combined effects. Further, by individually controlling each signal, the interaction of the signals may be fine-tuned and optimized.

The combined effects of applying the sonic signal and the EMF signal may include increasing the apparent saturation of dissolved species and enhancing the effectiveness of precipitating dissolved species from fluid sources. Without being bound by theory, sonic signals, when applied to a fluid, enhance the rate of nucleation and/or crystallization of dissolved species in the fluid (e.g., water). The sonic signal-generated pressure waves enhance the mixing of dissolved species within the fluid, increasing the frequency of collisions between ion pairs and the likelihood that a nucleation event will occur. Sonic signals may also cause sonocrystallation; sonofragmentation; sonochemistry; sonoluminiscense; fusion; microorganism inactivation; microorganism destruction; targeted biokill; initiation of selective chemical reaction; termination of selective chemical reaction; selective particle size crystallization and separation; establishment of conditions for high-energy physics; property alteration of the fluid, such as pH, concentration, temperature, pressure, suspended solids, dissolved solids, turbidity, viscosity, thermal and electric conductivity, density, surface tension, and other rheological and colligative properties; or a combination thereof. Similarly, EMF signals, when applied to a fluid can cause dissolved species to nucleate. The EMF signals interact with molecules in the fluid that possess dipole moments, such as water. For example, water molecules will be compelled to align with the EMF signal, disrupting the hydration layers around dissolved species that normally hinder the nucleation events. This effectively creates a temporary supersaturation state for the dissolved species. Without the hydration layer, counter-ions will be drawn together and nucleate, an event that would normally not occur until the saturation concentration had been reached. Additional ions will readily crystallize around this initial nucleus, removing the species from solution. Such nucleation may also have a biotoxic effect on microorganisms. Additionally, the combination of EMF signal and sonic signal may affect the localized ion distribution in the fluid changing colligative properties of the fluid, such as surface tension; thermodynamic properties, such as activity coefficients of the dissolved chemical species; transport properties, such heat, mass, and fluid and other factors that could affect the effectiveness of the fluid treatment.

Further, the combined effects of sonic signals and EMF signals include damage to microorganisms (e.g., death (e.g., via autolysis or necrosis), cell wall, damage, cell membrane damage, inhibition of growth, etc.). Without being bound by theory, when applied to a fluid, sonic signals create pressure waves in the fluid, which can cause acoustic cavitation. As the bubbles that are created collapse, high-velocity jets are emitted that may strike and damage the cell wall or cell membrane of a microorganism. The pressure waves created may also cause shearing forces strong enough to rupture a cell wall or cell membrane. The damaged microorganism, if able to survive, is then forced to expend energy repairing the damage instead of reproducing. The damaged cell wall or cell membrane is also unable to prevent chemical species from the surrounding fluid from entering the cell. Similarly, EMF is used in electroporation to introduce genetic material into cells by creating pores in the cell, membrane. When applied to a fluid containing a microorganism, EMF signals force phospholipids, which are dipole molecules, in the cell membrane to separate from each other, creating pores in the cell membrane. Under normal conditions, the cell will expend energy to close these pores once the EMF signal is removed. With constant application of EMF signals, the open pores in the cell membrane demand the microorganism to expend energy to attempt to repair the pores instead of reproducing. Additionally, the open pores in the cell membrane allow for the intrusion of active chemical species into the cell, eventually leading to the cell's death.

Additional combined effects of the application of a sonic signal and an EMF signal may include scale prevention, scale reduction, corrosion prevention, and corrosion reduction in structures carrying fluid that is being treated or that has been treated. The combination of sonic signals and EMF signals may enhance the previously identified physical phenomena of either signal operating alone. In other words, the combination of sonic signals and EMF signals may have synergistic results.

As noted above, systems of the present disclosure include a sonic energy generator that applies a sonic signal and an EMF generator that applies a conductive EMF signal to a fluid. In some embodiments, a system includes a vessel holding a fluid to which a sonic signal and a conductive electromagnetic field (EMF) signal is applied.

The systems and methods described herein may be used to treat water. In embodiments, such systems are used to treat water extracted from ambient air by an AWG unit. In such embodiments, the sonic energy generator and EMF generator may be located in any suitable location related to the AWG unit, e.g., coupled to a water storage tank, coupled to conduits that transfer the water from an AWG to a secondary water storage tank, coupled to a secondary water storage tank, and the like.

Embodiments disclosed herein also include systems comprising a plurality of AWG units in combination with at least one water treatment device that treats the extracted water with a sonic signal and a conductive EMF.

In order to describe particular embodiments of the devices and methods of the disclosure, reference is made to the appended figures. This discussion should not be construed as limiting, as the particular details of the embodiments described herein are by way of example and are for purposes of illustrative discussion of embodiments of the present disclosure. The particular embodiments described below refer to a sonicator as an example of a sonic energy generator, but the description below is not limited to a sonicator as a source of sonic energy.

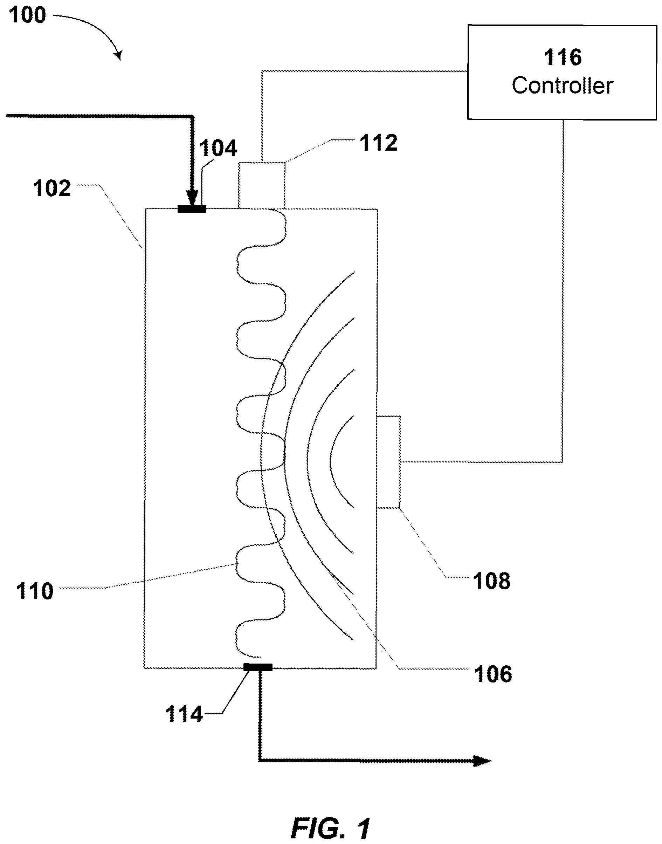

**FIG. 1** illustrates an embodiment of a fluid treatment system **100** that comprises a treatment vessel **102.** In embodiments, "treatment" when used with regard to a process or a related system for affecting the process, refers to a reduction in contaminants in a fluid by a statistically significant amount. In some embodiments, a treatment reduces contaminants in a fluid by at least about 20%. In further embodiments, a treatment reduces contaminants in a fluid by at least about 50%. In further embodiments, a treatment reduces contaminants in a fluid by at least about 70%. In further embodiments, a treatment reduces contaminants in a fluid by at least about 90%.

The directionality of the flow of the fluid that is to be treated is indicated by the arrows. The fluid enters the vessel **102** through a first port **104.** Although the fluid treatment system **100** depicts the treatment vessel **102** as a tank, treatment may be affected in a pipe or other structure in which fluids are stored or through which fluids travel.

While in the vessel **102,** the volume of fluid is treated with a sonic signal **106** produced by a sonicator **108** and a conductive EMF signal **110** produced by an EMF generator **112.** In some embodiments, the volume of fluid may be treated by a plurality of sonicators and/or a plurality of EMF generators. In some embodiments, the volume of fluid may be treated with a plurality of sonic signals and/or a plurality of EMF signals. In some embodiments, the volume of fluid may be treated through a plurality of vessels operated in series, parallel, or a combination thereof, within a larger system. In some such embodiments, each vessel is associated with a different sonicator and EMF generator.

Any suitable sonic energy generator, such as a sonicator may be used. In some embodiments, the sonicator **108** is an ultrasound generator. In other embodiments, the sonicator **108** is an energized antenna, a sonication horn, a hammer transducer, a sonic transducer, a magnetostrictive transducer, or a piezoelectric transducer.

A digital or analog function generator may be used to generate the sonic signal. Such a digital or analog function generator may include one or more amplitude, frequency, and/or phase modulators, or an arbitrary wave-form generator. A sonic signal **106** may be applied continuously or intermittently. In embodiments, the sonic signal **106** has a continuous waveform. In such embodiments, the waveform may be a sinusoidal waveform, a square waveform, a triangle waveform, or a sawtooth waveform. In embodiments, the waveform is a Dirac pulse form. In certain embodiments, the sonic signal **106** is a dampened waveform, e.g., a dampened sinusoidal waveform. In embodiments, the sonic signal **106** is pulsed regularly or randomly.

In embodiments, the sonic signal **106** causes bubble nucleation and cavitation in the volume of fluid in a stable manner, an inertial manner, or both. In some embodiments, the system may include other methods for bubble nucleation or formation, such as gas sparging, into the same volume of fluid.

The sonic signal **106** may have a fixed frequency or a variable frequency. The sonic signal **106** may be in the infrasound range (0 to 20 Hz), acoustic sound range (20 Hz to 20 kHz), the ultrasound range (20 kHz to 100 MHz), or above 100 Mhz. In some embodiments, the sonic signal **106** is an ultrasound signal. In embodiments, the frequency of the sonic signal **106** ranges from about 20 kHz to about 300 MHz. In some embodiments, the frequency of the sonic signal **106** ranges from about 20 kHz to about 200 MHz. In sortie embodiments, the frequency of the sonic signal **106** ranges from about 20 kHz to about 2 MHz. In certain embodiments, the frequency of the sonic signal **106** creates a resonant standing wave within the vessel **102.**

Any suitable EMF generator may be used (e.g., EMF generators as described in U.S. Pat. No. 9,181,113 and U.S. Patent Application 2016/0023926). Suitable EMF generators generate and apply an. EMF signal to the fluid via a direct conductive path (i.e., the transfer of electrical energy by means of physical contact via one or more conductive mediums) as opposed to via an inductive path. For example, the EMF generator **112** may include one or more contacts that transmit the conductive EMF signal **110** to the fluid. In embodiments, which include a vessel **102** including boundaries, e.g., walls, or portions of boundaries that are electrically conductive, the EMF signal **110** is transmitted through the fluid via the wall(s) of the vessel **102,** which are in electrical contact with the fluid. In other embodiments, the contact(s) are conductive elements (e.g., wire(s), mesh, projection(s), and the like) inside the vessel **102** where they can come into electrical contact with the fluid to be treated. The EMF generator **112** may be positioned on or near the exterior of the vessel **102,** or inside the vessel **102.**

The use of conductive EMF signals allows for a longer range and signal strength from more compact devices than inductive EMF devices. By using conductive EMF signals instead of inductive EMF signals, the same strength of signal may be produced with a lower power input.

A digital or analog function generator may be used to generate the EMF signal. Such a digital or analog function generator may include one or more amplitude, frequency, and/or phase modulators, or an arbitrary wave-form generator. An EMF signal **110** may be applied continuously or intermittently. In embodiments, the EMF signal **110** has a continuous waveform. In such embodiments, the waveform may be a sinusoidal waveform, a square waveform, a triangle waveform, or a sawtooth waveform. In embodiments, the waveform is a Dirac pulse form. In certain embodiments, the EMF signal **110** is a dampened waveform, e.g., a dampened sinusoidal waveform.

The EMF signal **110** waveform may have a fixed frequency or a variable frequency. In some embodiments, the frequency of the EMF signal **110** is randomized. In embodiments, the frequency of the EMF signal **110** ranges from about 0 kHz to about 100 MHz. In embodiments, the frequency of the EMF signal **110** ranges from about 10 kHz to about 500 kHz. In some embodiments, the frequency of the EMF signal **110** ranges from about 50 kHz to about 400 kHz. In some embodiments, the frequency, of the EMF signal **110** ranges from about 80 kHz to about 380 kHz. In some embodiments, the frequency of the EMF signal **110** ranges from about 1 kHz to about 80 MHz. In some embodiments, the frequency of the EMF signal **110** ranges from about 5 kHz to about 40 MHz.

In embodiments, the EMF signal **110** is pulsed regularly or randomly. In such embodiments, the EMF signal **110** may be pulsed at a frequency ranging from about 0 kHz to about 100 MHz. In some embodiments, the EMF signal **110** may be pulsed at a frequency ranging from about 1 kHz to about 80 kHz. In certain embodiments, the frequency of the EMF signal **110** ranges from about 5 kHz to about 40 MHz. In certain embodiments, the EMF signal **110** is a randomly-pulsed dampened sinusoidal waveform, with the waveform oscillation ranging from 80 kHz to 380 kHz and the pulsing frequency ranging from 5 kHz to 40 kHz.

The sonicator **108** and the EMF generator **112** may be controlled by a controller **116.** The controller **116** may independently control the sonicator **108** and the EMF generator **112.** In other words, the controller **116** may manage the generation and/or application of the sonic signal(s) and the EMF signal(s) independent of each other. In some embodiments, the controller **116** independently controls the sonic signal and the EMF signal, and monitors the combined application of the signals.

The EMF signal **110** and the sonic signal **106** may be synchronized, or substantially synchronized. Accordingly, in some embodiments, the EMF signal **110** and the sonic signal **106** are pulsed at substantially the same frequency. In embodiments, the EMF signal **110** is pulsed regularly at a frequency that is an integer value of a harmonic of the frequency of the sonic signal **106** that is at a higher or lower than the frequency of the sonic signal **106.** In some embodiments, the EMF signal **110** is phase-shifted from -180 degrees to 180 degrees from the sonic signal **106.** In some embodiments, the EMF signal **110** is phase-shifted from 0 degrees to 360 degrees from the sonic, signal **106.** In certain embodiments, the waveform of the sonic, signal **106** is a harmonic of the waveform of the EMF signal **110,** and is phase-shifted from -180 degrees to 180 degrees.

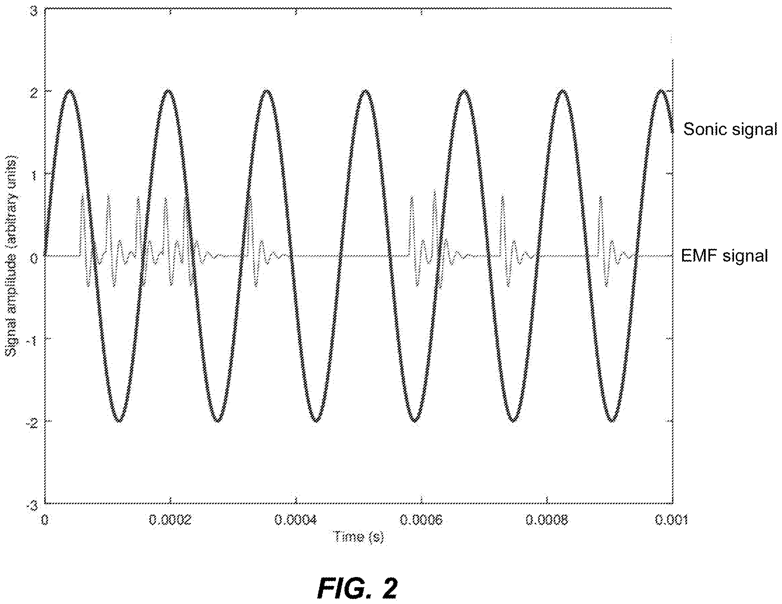

Examples of overlaid sonic signals **106** and EMF signals **110** are shown in **FIG. 2** through **FIG. 6****.** These figures show several graphs of a sonic signal (bolded line) and an EMF signal (unbolded line) on the same time axis. The sonic signal, represented by the bolded line, in each graph shows an example sinusoidal signal of 40 kHz. The EMF signal, represented by the unbolded line, shows various examples of signals that are based on a damped sinusoidal signal of 350 kHz. The amplitude of both signals is for illustrative purposes only. In some embodiments, the amplitude of the sonic signal is several times greater than the amplitude of the EMF signal. For purposes of this description, the positive amplitude of the sonic signal wave is taken to represent the compression portion of pressure waves, while the negative amplitude represents the expansion portion. These figures are presented as representative examples, and those skilled in the art will recognize that aspects such as waveform type, signal frequency, phase-shift, and EMF signal decay may be further varied within the scope of this disclosure.

In embodiments, the EMF signal is randomized, e.g., pulsed randomly (see, e.g., **FIG. 2**). As shown in **FIG. 2****,** the EMF signal is pulsed randomly, and is otherwise not correlated with the sonic signal. Such an EMF signal may be used in situations where resonance may occur and lead to ineffective EMF treatment, and, therefore, avoiding resonance is preferred. In such embodiments, a randomized EMF signal has a wide range of interactions with the sonic signal.

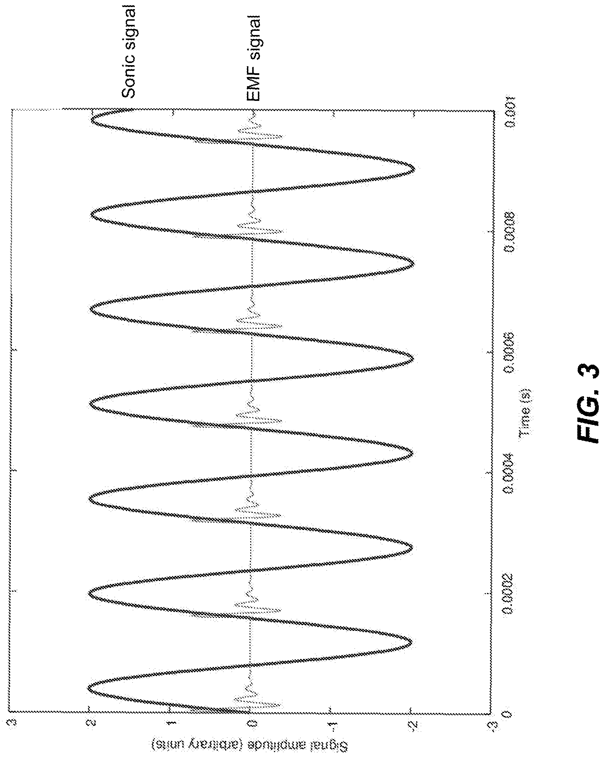

In some embodiments, the sonic signal and the EMF signal may be synchronized (see, e.g., **FIG. 3**). In some such embodiments, the sonic signal and the EMF signal are synchronized without any phase-shift. As shown in **FIG. 3****,** the EMF signal is pulsed at the same frequency (40 kHz) as the sonic signal, without any phase shift. In such embodiments, the EMF signal and sonic signal may have similar fluid treatment effects (e.g., homogeneous nucleation of supersaturated salts in water and damage to microorganisms). By timing the signals such that the EMF signal occurs at the onset of the compression wave of the sonic signal, the simultaneous waves can boost the effectiveness of the fluid treatment effects.

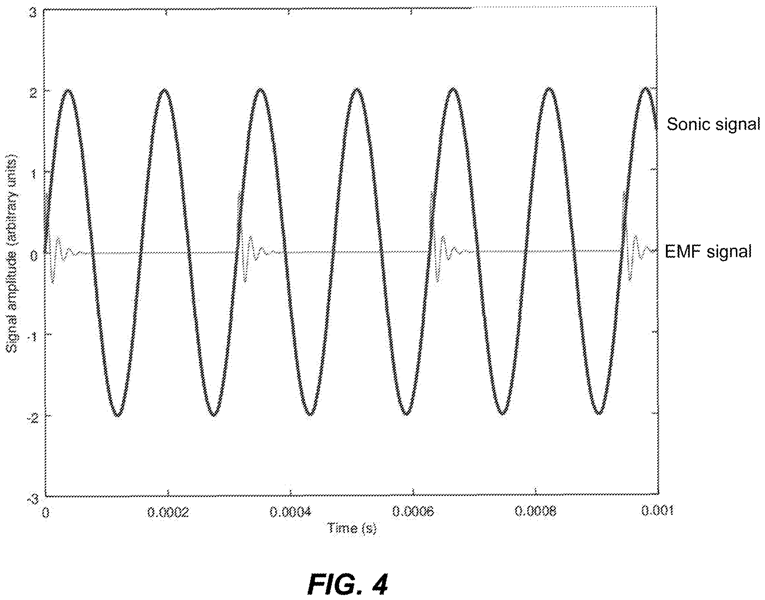

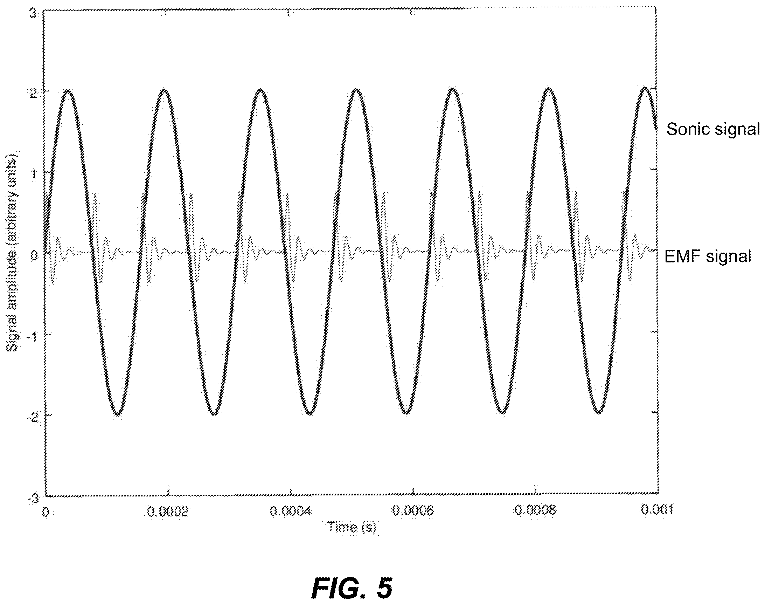

Synergistic effects can be achieved by synchronizing the EMF signal and the sonic signals, where one of the signals is a higher harmonic (i.e., has a frequency that is n times the frequency of the other signal, where n is an integer) than the other signal. For example, **FIG. 4** and **FIG. 5** demonstrate embodiments in which the EMF signal is pulsed at a lower harmonic frequency (20 kHz) and higher harmonic frequency (80 kHz), respectively, to the ultrasound signal. In both **FIG. 4** and **FIG. 5****,** the EMF signal and sonic signal are correlated to achieve similar simultaneous effects as described in the case of **FIG. 3** at certain cycles of the process. The signal correlation shown in **FIG. 4****,** where the EMF <signal pulsing is at lower frequencies, allows for a longer relaxation time of the hydration layers in the fluid as compared to the signals of **FIG. 3****.** This may be more effective to maximize the impact of the sonic signals in certain fluid conditions, for example, in removal of salts from highly-concentrated streams where only a small amount of supersaturation created by the EMF signal is required. The correlation shown in **FIG. 5****,** where the EMF pulsing is done more frequently, would significantly reduce that relaxation period, keeping the hydration layers of dissolved salts and the phospholipids in microorganism cells walls in constant flux as to make the sonic signal application more effective.

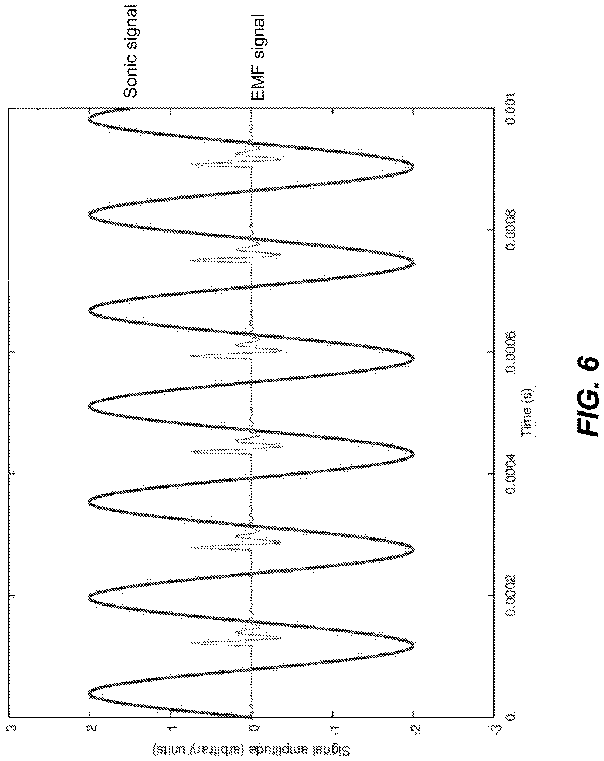

In some embodiments, the sonic signal and the EMF signal have the same frequency, but are offset from one another by a phase-shift. In **FIG. 6****,** the EMF signal is pulsed at the same frequency (40 kHz) as the sonic signal, but with a -90 degree phase shift from the sonic signal. In this scenario, starting the EMF signal prior to the compression portion of the sonic signal wave would allow for a few microseconds to disrupt hydration layers and cell walls or cell membranes prior to the impact of the compression wave, making the application of the sonic signal more effective at that time.

Returning to **FIG. 1****,** the sonic signal and EMF signal frequencies, amplitudes, waveforms, phase-shifts, and decay rates are all factors that would be adjusted based on the fluids that are to be treated and the contaminants in the fluid to be treated. In some embodiments, one or more of these values will be adjusted as necessary by the controller **116** for the sonicator **108** and EMF generator **112,** in response to a feedback loop. Such a feedback look may be built into a fluid treatment system **100.** For example, a sensor, e.g., a microorganism detector upstream of the vessel **102,** may observe an increase in the microorganism count upstream of the vessel **102** and transmit that reading to the controller **116.** The controller **116** may respond in one of several ways, such as increasing the sonic signal amplitude, increasing the EMF signal amplitude, adjusting the frequency of the sonic signal, adjusting the frequency of the EMF signal, altering the phase-shift between the EMF signal and the sonic signal, or a combination thereof, so as to achieve a correlated signal set that can best impact microorganisms at those levels. A similar feedback loop utilizing a sensor, e.g., a contaminant detector, can be utilized to achieve a correlated sonic and EMF signal set that can best impact the contaminants at those levels.

In some embodiments, signal processing of the sonic signal **106** and the EMF signal **110,** e.g., residual signal effects, such as a Doppler shift, resonance, and harmonics generated within the vessel **102** may be used, by the controller **116** to monitor, evaluate, or adjust the treatment conditions.

In embodiments, the controller **116** receives feedback from one or more sensors in real-time. In some embodiments, the sensor feedback is delayed or stored. In response to sensor feedback, the controller **116** may change a sonic signal feature, such as, frequency, intensity, and wave form; an EMF signal feature, such as, frequency, intensity, and wave form; and/or one or more synchronization parameters between the sonic and EMF signals, such as, frequency matching, harmonics, and phase shifting.

In embodiments, a fluid treatment system comprises more than one sonicator. Each sonicator may produce a sonic signal that has at least one characteristic (e.g., frequency, waveform, amplitude, phase-shift, decay, and the like) that is different than a sonic signal from another sonicator. In other embodiments, a sonicator produces a sonic signal that is substantially identical to a sonic signal from another sonicator in the system.

In embodiments, a fluid treatment system comprises more than one EMF generator. Each EMF generator may Produce an EMF signal that has at least one characteristic (e.g., frequency, waveform, amplitude, phase-shift, decay, and the like) that is different than an EMF signal from, another EMF generator. In other embodiments, an EMF generator produces an EMF signal that is substantially identical to an EMF signal from another EMF generator in the system. Although the EMF generator **112** in **FIG. 1** is shown coupled to the vessel **102,** an EMF generator may be located in any location that allows the EMF signal to be conductively applied to the volume of fluid, for example upstream of the vessel **102** (i.e., upstream of the first port **104**) or downstream of the vessel **102** (i.e., downstream of the second port **114**). In embodiments, an. EMF signal propagates through a portion of a vessel, such that the EMF signal interacts with at least one sonic signal. In some embodiments, an EMF signal propagates through the entire vessel.

The controller **116** may manage the sonic and/or EMF signal strength and frequency through the fluid treatment system in response to changes in upstream fluid conditions, downstream measurements of performance, or both. In various embodiments, controller **116** is a standalone system that controls sonicator **108** and EMF generator **112.** In some embodiments, controller **116** is part of an integrated control system for a larger fluid treatment system.

In embodiments that comprise more than one controller, the controllers can communicate with one another using a direct communication connection (either wired or wireless) or via a common network. In such embodiments, each controller can operate autonomously or can operate based on commands received from a central control station. In some embodiments, the central control station is in the same location as the controllers. In some embodiments, the central control station is located remotely. The common network may be any suitable network, such as an Ethernet network, a Modbus network, a CAN bus network, or some other appropriate communications network.

A controller may include a network communication device that can be connected to an external network. The external network can be a LAN, WAN, cloud, Internet, or some other network, and can be wired and/or wireless. Depending on the network, the protocol used can be any standard protocol, e.g., Ethernet, Modbus, CAN bus, TCP/IP, or any other appropriate protocol. Additionally, security and encryption technologies may be used.

The controller can be used to control and/or monitor the fluid treatment process via a graphical user interface (GUI). The GUI can include a display (e.g., an LCD, an LED display, etc.). In some embodiments, the display is integrated into the controller, or a remote GUI is attached to the controller via appropriate hardware.

A controller or a central control station can include a database for storing information, including, readings from sensors, flow rate data, power consumption data, and the like. Such a database can be stored in computer-readable media, on a separate device, or both. Along with the database, computer-readable media can also include the operating system and/or application software.

As shown in **FIG. 1****,** the treated fluid exits the vessel **102** through a second port **114,** such as an effluent port. In embodiments, a fluid treatment system, such as the one illustrated in **FIG. 1****,** is a standalone fluid treatment unit. In other embodiments, a fluid treatment system, such as the one illustrated in **FIG. 1****,** functions as a component of a larger fluid treatment system. In such embodiments, the treated fluid may undergo further treatments after exiting the vessel **102.** Further fluid treatment processes that may he employed include advanced oxidation processes (AOP; e.g., ultra-violet radiation, ozone treatment, hydrogen peroxide treatment, etc.); filtration (e.g., micro-, nano-, and ultra-filtration); reverse osmosis; forward osmosis; applied pressure; applied vacuum; mechanical agitation; gas sparging and degassing; thermal treatments, (e.g., multi-stage flash distillation); membrane distillation; electrodialysis; biological reactors; anaerobic and aerobic biological treatment; chemical treatment systems; treatment with nucleation agents; treatment with absorbents; treatment with adsorbents; treatment with a biocide; flocculation; electroflocculation systems; electrocoagulation systems; electromagnetic radiation; ion exchange columns or systems; treatment with a reducing agent, such as zero-valent iron; treatment with a catalyst; treatment with a photocatalyst; or a combination thereof. In some embodiments, an additional fluid treatment process comprises applying electromagnetic radiation; catalysts; photocatalysts; chemicals; biocides; nucleating agents; adsorbents; absorbents; thermal energy; biochemistry; or a combination thereof. In embodiments, a fluid treatment of the present disclosure may be combined with seawater/brackish water desalination, AWG, ocean thermal energy conversion (OTEC), tidal steam, energy generators, membrane bioreactors, or a combination thereof. In embodiments, the fluid to be treated includes drinking water, impaired water from agriculture, wastewater, ground water, surface water, grey water, seawater, produced water, flowback water, mining effluent, radiologically contaminated water, recycled water, industrial process water, cooling water, crude petroleum oil, processed petroleum, oil, a petroleum-based fuel, an organic solvent, a plant-based oil, a biofuel, a synthetic oil, a synthetic fuel, human physiological fluid, animal physiological fluid, or a combination thereof.

In embodiments, the fluid to be treated is water. In some embodiments, the fluid treatment systems may treat water for suitable purpose, such as, residential, industrial, indirect potable reuse, direct potable reuse (recycled water), ground water injection, commercial, food and beverage, hospitality, agricultural, mining, oil & gas, power, data centers, health-care, hospitals, nursing homes, pharmaceutical, government (e.g., military, federal, state, local, municipal, and foreign), security, space markets, emergency water use, fire-fighting use, or a combination thereof.

In some embodiments, the water to be treated is an impaired water stream that includes suspended solid materials. In such embodiments, the impaired water stream may include surface water, groundwater, black streams, grey water streams, agricultural wastewater streams, livestock wastewater streams, sludge, flowback and produced water streams from mining, drilling, and (racking operations, and wastewater effluent. In embodiments, the water contains at least 1 % suspended solids, by mass. In some embodiments, the water contains at least 5% suspended solids, by mass.

In embodiments, the fluid to be treated is an impaired water stream that includes suspended solid materials. In such embodiments, the water stream may be treated with an adsorbent solid material with large uptake capacities for specific contaminants in the water, such as zeolites, metals, metal oxides, activated carbon, molecular sieves, polymers, resins, clay, and the like. The water stream containing the adsorbent may then be treated with sonic signals and EMF signals. The sonic signals may help to loosen the solid materials, release gaseous and aqueous contaminants from the solids, and improve the rate and adsorption of these contaminants onto the adsorbent material.

The solids and adsorbent can then be recovered from the water through various processes, such as centrifugation and drying. By applying the sonic signals and EMF signals to the water stream, the water lost from the drying process is decreased, which also reduces the amount of energy required in such steps.

Additionally, applying the sonic signals and EMF signals to the water stream disrupts hydration layers on dissolved containments, reducing effective ion sizes and allowing the ions to enter smaller pores in the adsorbent material, and the signals prevent hydration layers from forming around ions as they enter the pores of the adsorbent material, enhancing the degree with which the ions can enter the pores of the adsorbent material. The fluid treatment systems described herein will produce separated water and solids that have lower levels of microorganisms (e.g., bacteria) than the starting impaired water stream, which may be, e.g., wastewater or animal waste effluent. After recovery, the solids may meet the standards for classification as Class A Biosolids by the Environmental Protection Agency (EPA).

In various embodiments, additional treatment processes may also be used before, after, or during the application of EMF signals and sonic signals. Additional treatment processes include, for example, adding chemicals for pH adjustment; flocculants; oxidizing agents, such as ozone, hydrogen peroxide, and ultra-violet radiation; reducing agents, such as zero-valent iron; catalysts; and the like. Such additional treatment processes may enhance the rate, capacity, or both, of contaminant removal from the impaired water. In embodiments, the EMF signals and sonic signals are applied to the impaired water while one or more adsorbent materials (e.g., metals, metal oxides, zeolites, activated carbon, molecular sieves, polymers, resins, and clays) are present.

In embodiments, the separated water then undergoes further treatment after the solids have been removed. In some embodiments, the water is treated by applying sonic signals and EMF signals from another sonicator and EMF generator, respectively. In some embodiments, the water is further treated with one or more additional treatment processes, such as filtration; reverse osmosis; forward osmosis; advanced oxidation treatments, such as ozone, hydrogen peroxide, and ultra-violet radiation; reducing agents, such as zero-valent iron; chemical treatment; electrochemical treatment; or a combination thereof.

In further embodiments, the fluid to be treated is water (e.g., surface water or ground water) contaminated with PFCs (e.g., perfluorinated alkyl halides, perfluorinated aryl halides, fluorchloroalkenes, perfluoroethers, perfluoroepoxides, pertluoroalcohols, pertluoroainines, perfluoroketones, perfluorocarboxylic acids, perfluoranitriles, perfluoroisonitriles, perfluorosulfonic acids, derivatives of perfluorosulfunic acids, and perfluorinated aryl borates). As is understood by those of skill in the art, PFCs, including pertluoroalkyl substances (PFASs), perfluorooctane sulfonate (PFOS), and perfluorooctanoic acid (PFOA), contaminate sources of drinking water and, unless removed, persist in the environment indefinitely.

In embodiments, adsorption by activated carbon is used to remove PFCs from water. By treating water contaminated by PFCs with activated carbon (e.g., granular activated carbon or powdered activated carbon) while applying EMF signals and sonic signals, the adsorption rate of the PFCs on the activated carbon, as well as the activated carbon's capacity, may be increased significantly. In embodiments, by applying EMF signals and sonic signals, the uptake of the PFCs to the activated carbon is increased by at least about 8 times, as compared to the uptake of PFCs to the activated carbon without application of EMF signals and sonic signals. In some embodiments, by applying EMF signals and sonic signals, the uptake of the PFCs to the activated carbon is increased by about 8 times to 10 times, as compared to the uptake of PFCs to the activated carbon without application of EMF signals and sonic signals. In various embodiments, additional treatment processes may also be used before, after, or during the application of EMF signals and sonic signals. Additional treatment processes include, for example, adding chemicals for pH adjustment; flocculants; oxidizing agents, such as ozone, hydrogen peroxide, and ultra-violet radiation; reducing agents, such as zero-valent iron; catalysts; and the like. Such additional treatment processes may enhance the rate, capacity, or both, of PFC removal from the impaired water.

In embodiments, EMF signals and sonic signals are used in conjunction with zeolites or ion exchange columns that utilize, e.g., a polymer or a polymeric resin, to remove PFCs from water. The polymeric resins of ion exchange columns may be provided as, e.g., small beads that may have similar mass diffusion limitations for PFC uptake as activated carbon. By treating water contaminated by PFCs with ion exchange columns while applying EMF signals and sonic signals, mass transfer limitations are improved. In embodiments, the adsorption rate of the PFCs on the ion exchange columns, as well as the ion exchange columns' capacity, may be increased significantly by combination exchange column treatment with EMF signals and sonic signals. In further embodiments, sonic signals and EMF signals are used to improve methods of regenerating the polymer or polymer resin material. The polymers or polymer resins are traditionally regenerated using a solution (e.g., a weak acid), which is later disposed of, to remove the PFCs. The additional of sonic signals and conductive EMF signals may increase the regeneration speed and the proportion of PFCs removed from the polymer or polymer resin materials. In embodiments, by applying EMF signals and sonic signals, the uptake of the PFCs to the ion exchange column is increased by at least about 8 times, as compared to the uptake of PFCs to the ion exchange column without application of EMF signals and sonic signals. In some embodiments, by applying EMF signals and sonic signals, the uptake of the PFCs to the ion exchange column is increased by about 8 times to 10 times, as compared to the uptake of PFCs to the ion exchange column without application of EMF signals and sonic signals.

In various embodiments, additional treatment processes may also be used before, after, or during the application of EMF signals and sonic signals to remove PFCs. Additional treatment processes include, for example, adding chemicals for pH adjustment; flocculants; oxidizing agents, such as ozone, hydrogen peroxide, and ultra-violet radiation; reducing agents, such as zero-valent iron; catalysts; and the like. Such additional treatment processes may enhance the rate, capacity, or both, of PFC removal, from the impaired water.

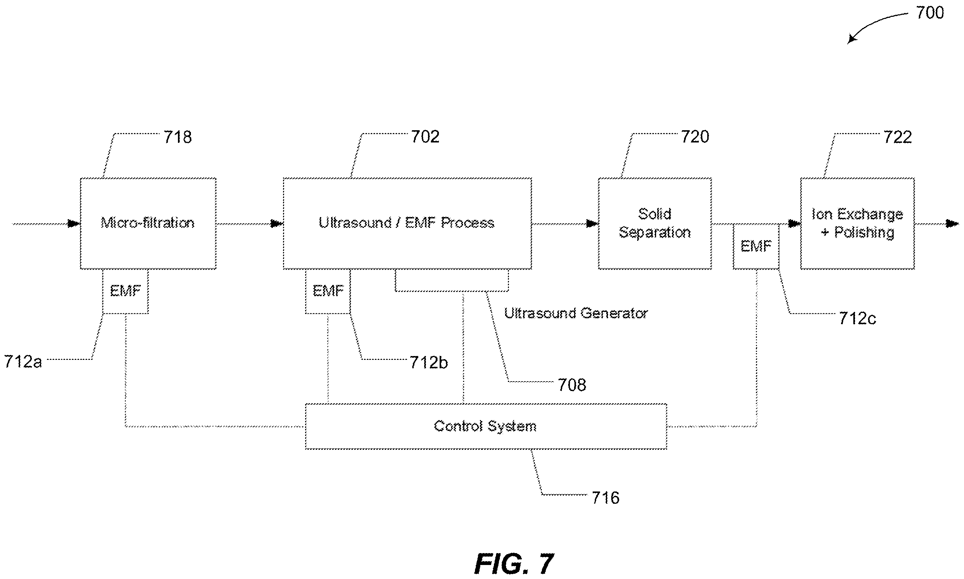

**FIG. 7** illustrates an embodiment of a larger fluid treatment system **700.** First, a fluid is passed through a micro-filtration membrane **718** while being treated with an EMF signal from EMF generator **712***a*. The fluid then travels to a vessel **702** in which the fluid is treated with an EMF signal >from EMF generator **712***b* and a sonic signal from sonicator **708,** as described above with regard to **FIG. 1****.**

The sonicator **708** and the EMF generators **712***a*, **712***b*, **712***c* may be controlled by a common controller 716. The controller **716** may independently control the sonicator **708** and each of the EMF generators **712, 712***b*, **712***c*. In other embodiments, not shown, each of the sonicator **708** and EMF generators **712***a*, **712***b*, **712***c* may be controlled by individual controllers. The controller **716** may manage the EMF signal strength and frequency in various locations (i.e., each of the EMF generators **712***a*, **712***b*, **712***c*) in of the fluid treatment system in response to changes in upstream fluid conditions, downstream measurements of performance, or both. In embodiments, the controller adjusts the EMF signal, the sonic signal, or both, in order to improve energy efficiency. In embodiments, the controller adjusts the EMF the sonic signal, or both, in order to adjust the quality of the resulting fluid.

After the fluid is treated with the sortie signal and EMF signal, the fluid is then transferred to a solid separation unit **720,** which may include one or more sedimentation steps, one or more filtration steps, one or more aeration steps, one or more dehydration steps, and the like. In embodiments, the water has less than 1 % by weight of suspended solids after being treated in the solid separation unit **720.** In some embodiments, the solids, after separation, are subject to a sterilization step. In some embodiments, the fluid may undergo a further sterilization step after the solids have been separated.

As shown in **FIG. 7****,** a third. EMF generator **712***c* is coupled to a conduit through which the fluid is transported from the solid separation unit **720** to an ion exchange and polishing tank **722.** As described above, the third EMF generator **712***c* applies an EMF signal to the fluid by a direct conductive path. The third EMF generator **712***c* may produce an. EMF signal that has at least one characteristic (e.g., frequency, waveform, amplitude, phase-shift, decay, and the like) that is different than an EMF signal from another EMF generator. In other embodiments, the third EMF generator **712***c* produces an EMF signal that is substantially identical to an EMF signal from another EMF generator in the system **700.** In further embodiments, multiple EMF units **712***a*, **712***b***, 712***c* may be used in a fluid treatment system without using a sonicator **708.**

In the ion exchange and polishing tank **722** the fluid is treated by removing one or more undesirable ionic contaminants by exchanging the undesirable ions with other less objectionable ions, as is understood by one of skill in the art.

In some embodiments, the controller **716** monitors the treatment process using one or more sensors, located throughout the system **700.** Such sensors may include, for example, sensors to measure temperature, pressure, conductivity, pH, turbidity, total dissolved solids, total suspended solids, biological oxygen demand, chemical oxygen demand, flow rate, chemical composition, microorganism counts, and the like.

In various embodiments, fluid treatment, systems (such as fluid treatment system **700**) comprise an additional fluid treatment process, such as advanced oxidation processes (AOP; e.g., ultra-violet radiation, ozone treatment, hydrogen peroxide treatment, etc.); filtration (e.g., micro-, nano-, and ultra-filtration); reverse osmosis; forward osmosis; thermal treatments, (e.g., multi-stage flash distillation); membrane electrodialysis; biological reactors; anaerobic, and aerobic biological treatment; chemical treatment systems, treatment with nucleation agents; treatment with absorbents; treatment with adsorbents; treatment with a biocide; flocculation; electroflocculation systems; electrocoagulation systems; electromagnetic radiation; ion exchange columns or systems; treatment with a catalyst; treatment with a photocatalyst; or a combination thereof. In some embodiments, an additional fluid treatment process comprises applying electromagnetic radiation, catalysts; photocatalysts; chemicals; biocides; nucleating agents; adsorbents; absorbents; thermal energy; biochemistry; or a combination thereof to the fluid. In, embodiments, an additional fluid treatment process may be mineralization, chlorination, addition of United. States Food and Drug Administration (US-FDA) approved flavor and taste additives, filtration (including micro-, ultra-, and nano-filtration), softening, dechlorination, deammonification, organic scavenging, deiozinzation, reverse osmosis, forward osmosis, distillation, ultra-violet radiation, sterilization, or a combination thereof.

The fluid treatment system may be organized in any suitable order. In embodiments, one or more additional fluid treatment processes may occur upstream, downstream, in the same vessel, or a combination thereof, as the applied sonic signal and EMF signal.

In embodiments, the fluid to be treated includes drinking water, impaired water from agriculture, wastewater, ground water, surface water, grey water, seawater, produced water, flowback water, mining effluent, radiologically contaminated water, recycled water, industrial process water, cooling water, crude petroleum oil, processed petroleum, oil, a petroleum-based fuel, an organic solvent, a plant-based oil, a biofuel, a synthetic oil, a synthetic fuel, human physiological fluid, animal physiological fluid, or a combination thereof.

In embodiments, the fluid to be treated is water. In some embodiments, the fluid treatment systems may treat water for suitable purpose, such as, residential, industrial, indirect potable reuse, direct potable reuse (recycled water), ground water injection, commercial, food & beverage, hospitality, agricultural, mining, oil & gas, power, data centers, health-care, hospitals, nursing homes, pharmaceutical, government (e.g., military, federal, state, local, municipal, and foreign), security, space markets, emergency water use, fire-fighting use, or a combination thereof.

In embodiments, the fluid to be treated is drinking water. In such embodiments, the water produced may meet purity standards from federal, state, and local laws for one or more of potable water, drinking water, bottled water, mineral water, high-performance water (pH>7.0), purified water (as defined by the US Pharmacopeia (USP), water for injection (USP), water for special pharmaceutical purposes (LISP), water for hemodialysis (LISP), sterile purified water (USP), non-potable water, secondary-treated water, tertiary-treated water, and recycled/reclaimed water. In some embodiments, the water produced may meet purity standards from federal, state, and local laws for one or more of groundwater reinjection, surface water injection, indirect potable reuse, direct potable reuse, aquifer recharge, and aquifer storage and recovery.

In embodiments, a fluid treatment system is coupled to an Atmospheric Water Generation (AWG) unit. AWG units draw in moisture-laden air and pass it through coolers and condenser systems to condense the water vapor into liquid water. The collected water generally has far less contamination as compared to surface or groundwater sources. In some embodiments, an AWG unit comprises one or more air filters that further decrease the levels of contaminants in the extracted water. Additionally, because AWG units can be operated without the need for water infrastructure, AWG units are ideal for providing water during emergency situations or where surface or groundwater is scarce. A fluid treatment system of the present disclosure would increase the amount of power needed by a relatively small amount as compared to the amount of power required to operate an AWG unit. Further, fluid treatment systems of the present disclosure do not require any additional chemicals to be added to the extracted water. Thus, an AWG unit, when combined with a fluid treatment system of the present disclosure, provides a source of water that has not been chemically treated. AWG units may include additional treatment systems that are used in combination with a fluid treatment system of the present disclosure (i.e., a system comprising a sonic energy generator and an EMF generator) to treat the collected water.

The controller of the fluid treatment system may adjust the treatment conditions based on readings from one or more sensors. Such sensors may include, for example, a humidity sensor, a temperature sensor, a pressure sensor, a wind speed sensor, or a combination thereof. In embodiments, a control unit of the AWG unit may act as a controller of the fluid treatment system. In various embodiments, a controller of the fluid treatment system may be integrated with a control unit of the AWG unit. In some embodiments, the fluid treatment system is active while the AWG unit is operating. In sortie embodiments, the fluid treatment system is active while the AWG unit is not operating.

Embodiments of the disclosure further comprise platforms that comprise one or more fluid treatment systems comprising a sonic energy generator and an EMF generator, as well as a variety of systems and subsystems (e.g., energy generation systems, energy storage systems, recycling systems, and the like). The various systems and subsystems may have individual control systems, may share a centralized control system, or both. The control systems may be structured in a system-of systems approach that uses data collection, aggregation, and analysis for optimizing the performance of each system of the platform in response to internal conditions, external conditions, or both. The control system may be used to monitor and control the entire platform, including, for example, activating or deactivating an individual unit in response to a change in, local weather.

In embodiments, the platforms of the disclosure are connected to one or more other devices via a network. In some embodiments, a control system of a platform of the disclosure is connected to one or more other devices via a network. In embodiments, the platform is connected to the Internet of Things (IoT). In some embodiments, a control system of a platform of the disclosure is connected to the IoT. In embodiments, data may be communicated using IoT protocols, such as Infrastructure (e.g., 6LowPAN, IPv4/IPv6, and RPL), Identification (e.g., EPC, uCode, IPv6, and URIs), Communications/Transport (e.g., Wifi, Bluetooth, and LPWAN), Discovery (e.g., Physical Web, niDNS, and DNS-SD), Data Protocols (e.g., MQTT, CORP, AMQP, Websocket, and Node), Device Management (e.g., TR-069 and OMA-DM), Semantic (e.g., JSON-LD and Web Thing Model), Multi-layer Frameworks, or a combination thereof.

An IoT-enabled control system may be in communication with an IoT system that tracks weather patterns at a larger scale (e.g., regional, state, national, etc.). In some embodiments, a control system, or a device connected to the control system via the network, uses data provided by the IoT system to model predicted weather conditions. In various embodiments, a control system may use data analysis tools, methods, and control strategies that include, for example, real-time model analysis; predictive modeling and control; artificial intelligence systems including, neural networks, fuzzy logic systems, genetic algorithms, and expert systems; software agents; knowledge management (KM) systems for data mining, cloud computing, parallel and distributed computing utilized in IoT for the fields of, e.g., Building Internet of Things (BloT), smart cities, infrastructure and utilities, healthcare systems, insurance industry, and manufacturing; or a combination thereof.

In some embodiments, a platform of the disclosure operates as a node within a nodal network of other water, power, and data generators and distributors, which includes data sharing throughout the nodal network to achieve optimum intra-platform and inter-platform performance for the entire nodal network capable of fault tolerant operations, addressing redundancy, resiliency, and stability of system operations during nominal, optimal, and emergency use.

The platforms of the, disclosure may include, one or more sensors that provide data to local control systems, as well as one or more networked devices. Such data may be used by the control system to optimize the performance of one or more systems. For example, a platform that comprises a plurality of AWG units may include a humidity sensor, or other sensor providing an indication of water vapor in the air, that provides readings to the control system. The humidity sensor may record an increase in humidity that causes the control system to activate one or more AWG units.

Platforms of the disclosure may comprise one or more systems that are configured to utilize a variety of components that are suited to the specific geographic region where the system is located. For example, a platform located near an ocean or salt-containing water source may include both AWG units, as well as reverse osmosis units and associated filtration systems to generate additional clean water from the ocean or salt-containing water source, and energy generation units to provide or offset electricity for the AWG units, osmosis units, and other components the platform. Additionally, the location of the system and the local and/or regional weather conditions (e.g., temperature, relative humidity, etc.) may be accounted for in real-time by the control system to respond to environmental changes, to negotiate with external factors (such as the BloT and IoT), or both. For example, a platform located near an ocean or salt-containing water source may also include temperature and humidity sensors as part of the control system. The readings from these sensors, through the control system, could be used to predict how much water the AWG units could produce, and to modulate how much water should be produced by the osmosis systems to augment the AWG water production, so as to maintain a continuous water production rate from the platform. This mode represents a type of on-demand water load-balancing. As another example, the platform could be built with deployable AWG units, and have a control system that communicates with external weather and water monitoring databases to detect events (e.g., natural or manmade emergencies) related to biosafety (e.g., hurricanes and flooding) and biosecurity (e.g., inadvertent or purposeful contamination of the water supply with toxic materials) of the water supply. If such an event is detected, the control system may begin procedures to deploy one or more AWG units. The controller would allow the AWG units to be disconnected from the platform and rapidly deployed to provide fresh water to affected persons during these emergencies.

In some embodiments, a platform of the disclosure is used to bring on new technologies alongside established technologies to provide for rapid prototyping and certification of new technology to meet the same federal, state, and local laws and regulations that the established platform technology must meet.

The platforms of the disclosure may include, or be located in close proximity to, one or more utilities. These utilities may be used to support the platform, provide access to the utilities to surrounding communities, support larger utilities, or a combination thereof.

In some embodiments, a platform includes a data/communications system. In some embodiments, a platform is located in close proximity to a data/communications system.

In embodiments, a platform includes a water treatment facility. In some embodiments, a platform is located in close proximity to a water treatment facility. In some such embodiments, the water treatment facility utilizes a surface water source.

In embodiments, a platform includes a power generation system or a power storage system (e.g. a solar cell system, a hydroelectric system, etc.). In some embodiments, a platform is located in close proximity to a power generation system or a power storage system. The power generation system or power storage system may be comprised of renewable components, non-renewable components, or a combination thereof. Suitable power generation or power storage systems include power plants based on fossil fuels (e.g., natural gas, coal, and oil), nuclear energy generated from subatomic particle reactions and collisions, renewable fuels (e.g., biofuels), or sustainable fuel stock (e.g., biomass and municipal solid waste/refuge-derived fuels). Additional power generation or power storage systems include renewable power systems, such as solar and wind farms; geothermal and hydroelectric systems; fuel cells; flow cells; solid-state, salt-based, and liquid battery systems; capacitors and ultra-capacitors; and thermal energy storage devices. In some embodiments, the platform is connected to conventional grid power. In other embodiments, the platform is not connected to the conventional power grid.

As described, above, the fluid treatment systems of the present disclosure may be used in combination with AWG units. In some embodiments, a platform of the present disclosure may comprise a plurality of AWG units and one or more fluid treatment systems. The plurality of AWG units may all be of the same type (e-g, manufacturer, capacity, etc.). In other embodiments, the plurality of AWG units comprises AWG units of more than, one type.

In some embodiments, one or more AWG units have a first setting and a second setting, the first setting having a higher extraction efficiency and a higher energy consumption compared to the second setting, and the second setting having a lower extraction efficiency and a lower energy consumption compared to the first setting. In embodiments, the first setting produces at least 5% more water at the same temperature and humidity conditions than the second setting. In some embodiments, the first setting uses at least 5% more energy at the same temperature and humidity conditions than the second setting.

In embodiments, an AWG unit may be operated in the first setting or the second setting based at least on data regarding geography, climate, weather, water demands or available supply, power demands or available supply, or a combination thereof. Such data may be received in real-time from a sensor, an external source, etc. or produced by predictive modeling. Sensors that may be of use include humidity sensor, a temperature sensor, a pressure sensor, a wind speed sensor, or a combination thereof.

The fluid treatment systems may be located in any suitable location, e.g., coupled to a co-located water storage tank, coupled to conduits that transfer the water from individual AWG units to a co-located water storage tank, coupled to a water storage tank for an individual AWG unit, and the like.

Each AWG unit may have an autonomous controller (e.g., an integrated controller for the AWG unit and the fluid treatment system) that may activate and deactivate the AWG unit without consideration of other AWG units. In such embodiments, the autonomous controller may activate or deactivate the AWG unit in order to maximize the performance of the AWG unit based on, for example, relative humidity and temperature of the surrounding air.

In other embodiments, more than one AWG units is controlled by a control system that activates and deactivates individual AWG units. In some such embodiments, the control system may change an AWG unit from a first setting to a second setting, or from a second setting to a first setting based at least on data regarding geography, climate, weather, water demands or available supply, power demands or available supply, or a combination thereof. The control system may be used to maximize the energy efficiency and water recovery of the plurality of AWG units as temperature and humidity change with time. In some, embodiments, the control system is used to maximize the water production rates, electrical and water recovery efficiencies while minimizing power usage and energy and water production costs.

Additionally, the control system may be used to control the fluid treatment system of the present disclosure, including at least one sonic energy generator and at least one EMF generator, as well as any additional treatment systems.

In further embodiments, a platform of the present disclosure may serve as a source for water and power in an emergency (e.g., for disaster response) or for locations in which it is preferable to not utilize conventional water or power. In such situations, platforms of the present disclosure may provide water resiliency (i.e., the ability to resist, absorb, accommodate to, and/or recover from the effects of a hazard in a timely and efficient manner) and water security. Such a platform may'be configured as a satellite emergency response system for water treatment, power, and data communications to be deployed for emergency response and use. In various embodiments, a platform is configured to meet military-grade command, control, communication and computer (C4) operations for US Department of Defense (DoD) and Department of Homeland Security (DHS), as well as International Security Organizations (ISO). In such embodiments, the platform may be IoT enabled with necessary protocols to operate within DoD, DHS, and ISO specifications.

In some embodiments, one or more subsystems of the platform are not locationally fixed, and may be moved to another location in an expeditionary, emergency, and/or remote mode. In some such embodiments, each sub-system may be operated in a temporary, intermittent, and/or permanent scheme.

In embodiments, the platform operates as a trading desk for water, power, and data commodities trading (i.e., transactions for buying and selling of water, power, and associated data). The water, power, and associated data may be sold for its highest and best use, or for maximum value based on the availability of local water and power usage. In embodiments, the water and power generated are sold to customers when these commodities are not readily available from local municipalities or other local sources, if it is not cost, effective to purchase the commodities from, such sources, or both. Such trading desks can be used as a virtual support of local future markets, suited for diurnal (e.g., daytime, when the sun is available and at night when it is not that effects variables such as incident light, heat, temperature, relative humidity, pressure, wind speeds, etc.) and seasonal conditions, as well as for local spot market sales of water, power, and related data. The platform may be arranged as an optimal network of systems to generate, store, negotiate sales of, and distribute water, power, and related data. The optimal network of systems may also serve as a local resource to further communicate and negotiate specific outcomes with the loT.

In some embodiments, the platform (1) collects and aggregates data from one or more internal or external data sources, including one or more elements selected from the group consisting of: historical data; past and current operating, conditions of the platform's systems and sub-systems; current environmental and weather conditions; external databases and information sources such as National Oceanic and Atmospheric Administration (NOVA), Geographic Information Systems (GIS), and United State Geological Survey (USGS) databases; global, federal, state, and local databases and information sources such as DHS, EPA, and the United States Departments of Ecology and Energy; signaling and events from external water, power, and data generators and distribution systems; past and current performance, efficiencies, and quality of water, power, and data produced by the platform; past, current, and projected external conditions; model estimates and predictions; and past, current, and future supply and demand for water, power, and data; (2) defines a set of candidate operating parameters for the platform that defines the source(s) of water, power, and data; the target quantity and quality of water, power, and data to be generated; the control of the distribution of water, power, and data; signals and events to be sent to external water, power, and data generators and distribution systems; and other associated operating conditions for the platform; (3) uses an optimization function to evaluate the candidate operating parameters against the aggregated data; (4) adjusts or refines the candidate operating parameters to minimize or maximize the optimization function through an optimization search algorithm, with consideration of federal, state, or local laws and regulations, or system or sub-system limitations; and (5) applies the selected operating parameters to the platform.

In further embodiments, the platform may be used to support a system of water use efficiency. In such embodiments, the optimization of water, power, and data generation may be used to support such a system for water use efficiency. Such embodiments include a method in which water produced by a platform is valued proportional to the volume of potable (primary), secondary, and tertiary water that is generated and made available for distribution. In some embodiments, a system or subsystem of the platform analyzes and negotiates for water available for immediate use and/or generated water based on immediate, mid-term, and long-term demand based on the most efficient source of water to parties (e.g., wholesalers, retailers and end-users) as potable, secondary; and tertiary water sources. In some embodiments, a system or subsystem of the platform determines and transmits a signal to another platform or an external device to recommend water use reduction and conservation, beneficial uses of water, water recycling and reuse, and accessing water of alternative water sources. In some such embodiments, a system or subsystem of the platform determines and transmits a suggested setting for one or more AWG units to increase water production, reduce power usage, etc. Accordingly, embodiments include optimization of water, power, and data generation is used to support a system for water use efficiency, which includes; (1) methods, by which water producers could value, or be allocated the agreed upon value, of water proportional to the volume of potable (primary), secondary, and tertiary water they generate and make available for distribution; (2) methods by which a platform of the disclosure can analyze and negotiate between water available for immediate use and/or generate water based upon the immediate, mid-term, and long-term demand based on the most efficient method or source of water to wholesalers, retailers, and end-users as potable, secondary, and tertiary water sources; and/or (3) methods to determine and transmit appropriate signals and suggested modes to a platform of the disclosure and/or a network of platforms to recommend water use reduction and conservation, beneficial uses of water, water recycling and reuse, and accessing water of alternative water sources.

Platforms of the disclosure may incorporate an environmental management system (EMS) framework that defines a set of processes and practices for reducing the environmental impact of the platform, and for meeting federal, state, and local laws and regulations for environmental impact and industry-established standards (such as ISO 14001), by integrating the data, and communication systems on the platform with additional internal and external data, software, and systems. In some embodiments, the EMS includes environmental compliance, health and safety compliance; energy efficiency; energy conservation; water conservation; environmental conservation; environmental sustainability; waste reduction; hazardous materials and waste management systems; utility management systems; planning, programming, budgeting and execution systems; workflow management systems; training and work practices documentation and systems; maintenance and support systems; risk assessment and management systems; emergency preparedness and response systems; physical and electronic security software and systems; EMS data tracking and monitoring software and systems; EMS data analysis software and systems; EMS Big Data and data analytics software and systems; EMS reporting software and systems; EMS interface software and systems; EMS auditing software and systems; compliance assessment and reporting software and systems; or a combination thereof. In some such embodiments, the EMS descriptive and prescriptive analysis is a result of the platform's EMS data analytics, auditing, and compliance assessment provides the necessary documentation to demonstrate lowest risk and warrant lower insurance premiums.

In further embodiments, the EMS includes water management plan (WMP) to outline descriptive and prescriptive steps and control measures to assure the microbiological quality of water (such as detection of *E. Coli* and Legionella) produced by the platform to meet federal, state, and local water quality requirements and industry-established standards (such as ASHRAE 188). In some embodiments, the EMS and the platform(s) may be used to rapidly on-board and update all stakeholders to the physical platform or network configuration and layout (such as process flow diagrams (PFD), process instrumentation drawing (P&ID) and water and power distribution systems), performance metrics, preventative verification, control measures, validation, and associated documentation, utility and commodity consequences to financial outcomes (such as financial statements and strategies as they relate to business operation and the trading desk operations described above) of all processes as part of a training methodology to provide platform-relevant skills and information to appropriate stakeholders. In some embodiments, the platform-relevant operation and maintenance skills include, e.g., water treatment, water generation, energy generation, energy efficiency, energy conservation, environmental sustainability, environmental, conservation, or a combination thereof.

Platforms that comprise a plurality of AWG units that is collectively capable of producing at least 10,000 gallons per day are considered large scale AWG platforms. In embodiments, such platforms comprise more than one type of AWG unit. In some embodiments, the types of AWG units may have varying efficiency based on the relative humidity. In embodiments, the first type produces at least 5% more water at the optimal temperature and humidity conditions than the second type. In embodiments, the electrical efficiency of the first type is at least 5% different at the optimal temperature and humidity conditions than the second type.

Within a large scale AWG platform, the selection, design, and relative capacity of the various AWG units would be highly dependent on the location and user-requirements for the platform.

A control system of the large scale AWG platform may activate and deactivate one or more AWG units of a particular type depending on the conditions (e.g., temperature, humidity, wind speed, wind direction, etc.) in order to maximize energy efficiency and water recovery of the large scale AWG platform. In various embodiments, one or more AWG units of a particular type are activated or deactivated based on a relative humidity (RH) threshold value (i.e., an RH trigger value). The RH trigger value may be based on the energy efficiency of an AWG unit at a particular RH value. The RH trigger value can be optimized to improve electrical efficiency within the large scale AWG platform. Activating AWG units that are electrically inefficient at a low RH, but very electrically efficient at a high RH, at too low of an RH trigger value may lead to poor overall electrical efficiency of the platform. On the other hand, activating the same AWG units at too high of an RH trigger value may significantly lower the overall efficiency of the platform by failing to utilize the improved individual efficiency of the AWG units at high humidity.

The analysis of the optimum RH trigger value would be different for each large scale AWG platform, and would vary based on location, weather patterns, end-user requirements, and the like. Such an analysis would be platform specific and may be based on performance curves for specific types of AWG units, historical weather data from the location, and the like. Similarly, the analysis to determine the optimum ratio of types of AWG units that would provide optimum electrical efficiency for a given RH trigger value, will take into consideration their respective capital and operating costs. An example of such an analysis is provided as Example 1.

The current weather in a given location, as well as the predicted weather, may impact the ratio and number of AWG units of varying types that are activated or deactivated. For example, if a pressure front is forecasted to move over the location, and precipitation is expected, which is a sign of high humidity, then the control system may activate a type of AWG units earlier, or more AWG units of that type than would otherwise be activated, to increase water recovery, electrical efficiency, or both.

In embodiments, a large scale AWG platform is operated to optimize the water production efficiency, power production efficiency, and costs for each AWG unit and for the large scale AWG platform as a whole. In some embodiments, a large scale AWG platform is also operated to optimize the water production efficiency, power production efficiency, and costs for a plurality of large scale AWG platforms. In some such embodiments, the manner of operation (e.g., number of activated AWG units, ratio of AWG units of varying types that are activated, etc.) is based on historical data, real-time data, model estimates, and/or projections of water, power, and data generated and consumption. Such manners of operation may create an ad hoc network of satellite utility platform installations that interacts, supports, sand augments traditional utility installations. In embodiments, the manner of operation of the plurality of large scale AWG platforms is determined by (1) collecting and aggregating data from one or more internal or external data sources, e.g., historical data; past and current operating conditions of the platform's systems and sub-systems; past and current performance; efficiencies, and quality of water, power, and data produced by the platforms within, past, current, and projected external conditions; model estimates and predictions; and current arid future supply and demand for water, power, and data; (2) defining a set of candidate operating parameters for each platform within the network that defines the source(s) of water, power and data, the target quantity and quality of water, power, and data to be generated, and other associated operating conditions for the platform; (3) using an optimization function to evaluate the candidate operating parameters against the aggregated data; (4) adjusting or refining the candidate operating parameters to minimize or maximize the optimization function through an optimization search algorithm, with consideration of federal, state, or local laws and regulations, or system or sub-system limitations; and (5) applying the selected operating parameters to the platforms on the network.

A large scale AWG platform is flexible and can access sources of water found in the atmosphere, in or on land, in the ocean, or in other brackish bodies of water. For example, a location may have access to an impaired water source from a well, aquifer, lake, reservoir, or municipal treatment plant that is not operating to public drinking standards, is unreliable, or has tainted water from the distribution infrastructure. A large scale AWG platform could be installed in this location that could address the impaired available sources via the fluid treatment using sonic signals and. EMF signals of the present disclosure, in combination with a plurality of AWG units to extract additional water from the atmosphere for potable and non-potable use. The large scale AWG platform could provide an immediate source of clean water from atmosphere via the plurality of AWG units, while the water treatment system processes the water collected from the other sources.

In some embodiments, a large scale AWG platform is located near a body of water, water treatment, power production, or a combination thereof. A large scale AWG platform may be situated near an existing natural or man-made body of water. In some embodiments, the AWG units are located principally downwind of an existing water source (e.g., ocean, sea, lake, or river). In some embodiments, an AWG unit is located within one mile of a body of water. Alternatively or additionally, the large scale AWG platform site may include man-made open water pools, such as retention ponds, that are located so that the AWG units of the platform sit downwind from the water sources. In such embodiments, the wind passing over the bodies of water may accumulate additional moisture drawn into the air by natural evaporation prior to reaching the AWG units, and increases the moisture that may be collected by the large scale AWG platform. The water source(s) may be impure, such as surface water, including fresh and sea water, rain water ponds, or wastewater tanks. The evaporated moisture will be primarily clean water, with the contaminants being left behind. The. AWG units and the fluid treatment systems of the present disclosure provide a further level of protection in the produced water purity.

A large scale AWG platform may be located near water sources that are either a part of, or combined with, another water treatment system, such as wastewater treatment plants (e.g., municipal wastewater treatment plants, reclaimed water treatment plants, etc.), brackish/seawater desalination plants, OTEC, tidal steam energy generators, geothermal systems, hydroelectric plants, and remediation ponds for drilling, mining, and hydraulic fracturing water treatment. Such water treatment plants generally consume a large amount of power and may be co-located with power plants. Accordingly, in some embodiments, a large scale AWG platform is co-located with a power plant. The large scale AWG platform can be designed to work within the specifications of the existing power supply, and use the open water sources in the water treatment site to gain additional moisture from which clean water may be extracted. In some embodiments, the large scale AWG platform is used to augment water production from the co-located water treatment or production facility. For example, the co-located system may be a desalination plant with an optional power generation, and the large, scale AWG platform may be used to further treat waste water rejected by the desalination plant. In some embodiments, the large scale AWG platform uses the co-located water treatment or production facility to provide additional water to reach a minimum water production rate when the AWG units or other water treatment systems on the platform temporarily cannot meet the minimum, production rate.

In embodiments, a well-designed configuration or physical arrangement of the AWG units in a large scale AWG platform would be one that assures good air distribution between and around the units, so that all units would be drawing from air with substantially the same moisture content. In embodiments, the AWG units of a large scale AWG platform may be installed in a three-dimensional pattern in an open-air framework. Such an arrangement may increase the amount of moisture captured from the air by extracting water from air that is further from the ground. Further, such an arrangement may improve efficiency and reduce the land footprint or area required, for example, a multi-storied open structure, similar to an open-air parking garage, with sufficient clearance between each story for the AWG units, could be used. Other structures such as ramps, e.g., a helical ramp, may also be used. In additional examples, AWG units are positioned atop existing structures. Accordingly, in some embodiments, the arrangement of the AWG units of a large scale AWG platform includes the elevation and the geographic position of each unit.

Evaporative processes may also be utilized in a "bio-sphere" or closed environment. Such a large scale AWG platform design may be more effective when the motion of the air is accelerated through the site. Faster air motion may increase rates of mass transport and evaporation across an open surface, which increases the moisture concentration, i.e., humidity, of the air.

In some embodiments, the AWG units of a large scale AWG platform are, arranged such that each AWG unit is drawing from air that has a temperature and moisture content that is within +/-2% of the air's temperature and moisture content upwind of the large scale AWG platform, based on historical and model averages of the weather behavior around the site. In some embodiments, the configuration of the AWG units of a large scale AWG platform is mathematical modeled to determine the optimal placement of each AWG unit such that the inlet air temperature and moisture content is normalized for each AWG unit, on average.

In embodiments, additional fixtures designed to direct and accelerate air flow are arranged in locations adjacent to AWG units. In some such embodiments, the additional fixtures may be arranged upwind of the AWG units. Such additional fixtures may be arranged in order to increase the moisture content of the air surrounding the AWG units. Any suitable additional fixtures may be used, including, passive units (e.g., walls, columns, blades, baffles, grates or other hardened structures) or powered units (e.g., motorized fans or venturi effect-based fans). In embodiments where the fixtures are powered units, the units may be controlled to adjust the air speed over the AWG unit. These fixtures may be mounted on bases that rotate with the prevailing wind direction. In some embodiments, the fixtures are mounted on bases that are controlled. In such embodiments, the fixtures may be turned, either automatically or manually, with changes in the wind direction in order to increase the air velocity and mixing at or near the AWG unit location.

In some embodiments an additional structure, such as a windmill or a wind turbine, is located downwind of the AWG units in order to recover momentum energy from vented air from the AWG units.

In embodiments, the arrangement of AWG units in a large scale AWG platform are chosen so as to not disrupt atmospheric weather conditions via dehumidification of downwind locations.

As all AWG units in the large scale AWG platform are drawing moisture from the ambient air, AWG units located downwind of other AWG units may have lower performance, as the air may have a lower moisture content. Similarly, the location of a large scale AWG platform relative to another large scale AWG platform may impact the efficiency and capacity for each platform. In some embodiments, the arrangement of AWG units in a large scale AWG platform is chosen so as not to be adversely impacted by, a second large scale AWG platform that is located upwind.

In embodiments, the location of an AWG unit relative to natural or man-made features, the configuration and arrangement of an AWG unit within a large scale AWG platform, and the placement of a large scale AWG platform relative to other large-scale AWG platforms, are selected to optimize the overall water production capacity and energy efficiency of the large scale AWG platform.

Studies have shown that large-scale renewable power systems, such as wind and solar, may cause small, but significant, changes in weather due to their impact on momentum and energy in the atmosphere. Similarly, large scale AWG platforms may in the same, or a similar, manner by impact the moisture;content of the ambient air. In various embodiments, a large scale AWG platform does not have a significant impact CM a Natural Local Atmosphere. The "Natural Local Atmosphere" (NLA) refers to a specific area, domain, and'or volume directly above the Earth's surface without the influence on the local atmosphere by any large scale AWG platform. In embodiments, the atmospheric ceiling, is at least 3,000 meters. In some embodiments, the atmospheric ceiling is from 100 meters to 10,000 meters. In some embodiments, the atmospheric ceiling is from 1,000 meters to 5,000 meters.

The air in a NLA will have the maximum moisture content that is available to be extracted by a large scale AWG platform. In some embodiments, the production rate of an AWG unit will be highest when operating in a NLA.

In some embodiments, AWG units of a large scale AWG platform are arranged to maximize the moisture content of the ambient air and production rate for a given location and AWG unit efficiency given the local weather patterns. In some embodiments, two or more large scale AWG platforms are arranged to maximize the moisture content of the ambient air and production rate for a given location and AWG unit efficiency given the local weather patterns. In some embodiments, the separation of each large scale AWG platform is at least one mile. In some embodiments, a large scale AWG platform is placed at a site located between the latitudes of North 35° to South 35° (i.e., in the tropics and subtropics). In certain embodiments, a large scale AWG platform is placed at a site located between the latitudes of North 23.5° and 35° or South 23.5° and 35° (i.e., in the subtropics).

The optimal arrangement of one, or more large scale AWG platforms may be calculated in any suitable manner. In some embodiments, an initial placement for the one or more large scale AWG platforms is chosen; mathematical model(s) are used to determine the NLA and the large scale AWG atmosphere (LSAWGA) conditions for each large scale AWG platform; and this information is compared to additional data sources to determine if the proposed locations are acceptable. If not, the placements may be refined. The models used to determine. NLA and LSAWGA may incorporate temperature, pressure, humidity, wind speed and wind direction, precipitation, cloud cover, solar radiation, air composition, pollution, time of day, time of year, geographic coordinates, land elevation data, land use data, vegetation coverage, urban area coverage (cities, building sizes and types), soil types, the arrangement Large-Scale AWG platform, or a combination thereof. In some, embodiments, the calculation of NLA and LSAWGA incorporates historical and predicted weather and climate data; mathematic weather prediction models; geographic data sources such as GIS or ARCInfo; water source data such as USGS data; and/or local water, power, and data utility capacity and generation/distribution.

For example, in order to determine an, optimal arrangement of one or more large scale AWG platforms (N=number of large scale AWG platforms, where N≥1) in a geographic region, where the distance from the site to the atmosphere to the maximum elevation Z ₘₐₓ (vertical; z-variable) has a value of at least 3,000 meters. The optimal arrangement, for purposes of this example, is to place the one or more large scale AWG platforms in the smallest possible area without changing the NLA. In other words, the one or more large scale AWG platforms would be located in the smallest geographic area that enables the optimum water to be extracted from the atmosphere to a maximum elevation (zₘₐₓ) from all large scale AWG platforms. Therefore, the minimum distance, (x, y) variables, that a large scale AWG platform can be placed in proximity to another large scale AWG platform without changing the NLA will be determined. The planned placement of each large scale AWG platform is defined by coordinates (x₁, y₁), (x₂, y₂), ... (x_{N}, y_{N}). The NLA for the region is determined based on historical weather patterns, and includes atmospheric properties such as moisture M (kg/kg), defined at each point within the region. For example, M_{NLA} (x₁, y₁) represents the moisture content of the NLA at the planned site to zₘₐₓ in elevation for the first large scale AWG platform. A similar method is used to determine the predicted atmospheric properties for the placement of all additional large scale AWG platform, which is LSAWGA. LSAWGA₁ represents the predicted atmosphere conditions with the addition of the first large scale AWG platform, LSAWGA₂, represents the predicted atmosphere conditions with the addition of the second large scale AWG platform (in addition to the first large scale AWG platform), and so forth. Thus, the moisture content at the first large scale AWG platform is M_{(LSAWGA1)} (x₁, y₁). By determining the conditions of the atmosphere, LSAWGA, with the addition of each large scale AWG platform, the placement of the large scale AWG platform that would maintain the NLA may be determined. In some embodiments, the comparison between NLA and. LSAWGA is made using an objective function, which may be resolved spatially and/or temporally, applied to NLA and LSAWGA that includes the evaluation of one or more variables (including their average values, their standard deviations, and/or their variables) over at least some of the volume within the region, and/or over one or more time segments. In such embodiments, variables that may be incorporated into the objective function include temperature; pressure; humidity; wind speed; wind direction; turbulence measurements; precipitation; weather fronts; weather storm systems (such as tornados, hurricanes, and typhoons); cloud cover; surface parameterization parameters; buoyancy stability; inertial stability; Monin-Obukhov length; bulk Richardson number; gradient Richardson number; planetary boundary layer height; momentum surface roughness length; heat surface roughness length; moisture surface roughness length; surface fluxes for momentum, latent heat, and moisture; hydrology parameters; water table levels; surface water locations, capacity, and current levels; groundwater levels; aquifer capacities and current levels; surface water quality; groundwater quality; ocean currents; ocean tides; air-to-sea interface parameters; water demand requirements; electrical demand requirements; water usage data; power usage data; electricity costs; water utility costs; land elevation; land use; land costs; capital equipment costs; operating and maintenance costs; population; population growth; short- and long-term climate forecasting, long-term water availability; and/or drought condition estimates.

Such methods of arranging the AWG units of a large scale AWG platform may be used to influence the local weather or to collect water from the air, as a type of atmospheric water harvesting. In such embodiments, a large scale AWG platform may be used to extract layers of water, held in water-laden air, as an atmospheric resource. In some embodiments, a large scale AWG platform may be used to direct water-laden air to a second large scale AWG platform.

As is understood by one of skill in the art, large natural features, such as mountains, hills, lakes, oceans, and vegetation may impact the flow of air (e.g., wind patterns) at a local scale. For example, humid warm air that is pushed over a mountain will likely condense as rain as the air cools due to the higher elevation of the water-laden air mass. Using similar principles, a large scale AWG platform may be located in order to create local weather lanes. In some embodiments, a large scale AWG platform may be arranged to create a local weather pattern berm. Large scale AWG platforms may be arranged and used to deliberately influence weather patterns (e.g., short-term weather patterns) into a beneficial use, such as by removing moisture from the air to help reduce the instability of the air as a type of weather buffer, or by using other nearby geographic features to promote a general trend fore wind flow direction. The effects of a large scale AWG platform on regional weather patterns have been modeled, and the results are described in Example 2.

Although the specific configuration of a large scale AWG platform will vary depending on location, end-goals, weather patterns, etc., the method of arranging the AWG units described herein may be applied.

### EXAMPLE 1

A large scale AWG platform includes a plurality of small AWG units and a plurality of large AWG units. The small AWG units are efficient at high humidity levels (e.g., 80% or better relative humidity), but are vow inefficient at low humidity levels (e.g., below 50% relative humidity). The large AWG units are not as efficient at high humidity as the small units, but can generate appreciable amounts of water at a relative humidity lower than 50%. In such case, the control system may operate the large units continuously in order to base-load the generation of water, while activating the small units when the humidity exceeds 50% to gain the advantage of their efficiency at this point. Depending on several factors (e.g., power costs, etc.), the control system may activate the small units when the humidity exceeds 80%. By utilizing a combination of AWG units, the control system is able to maximize the generation of water while minimizing the energy required, as compared to using strictly one type of AWG unit or the other.

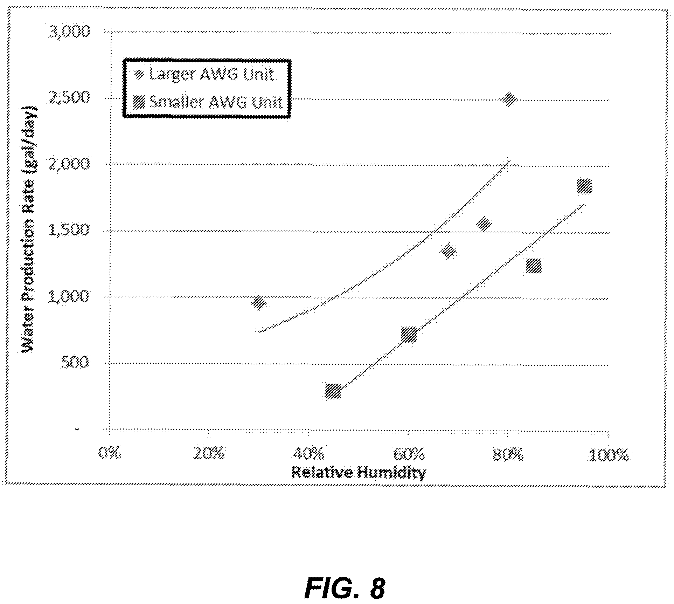

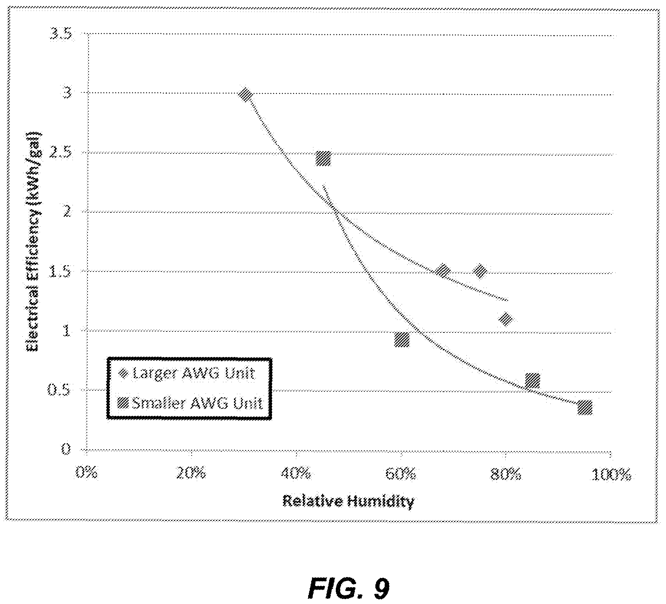

To further illustrate this example, the reported performance of two commercial AWG units was used to model a large scale AWG platform. The large. AWG unit used in the model is optimized to generate 2,500 gal/day of water, at 80% relative humidity (RH) and can readily handle low humidity. The small AWG is optimized to generate 1-250 gal/day at 80% RH with good electrical efficiency, but does not perform well at humidity less than 50%. The humidity is the largest driver for the AWG unit performance. Although temperature may play a significant role in the performance for an AWG unit, for purposes of this example we assume that the temperature effects are equivalent for both units and do not account for them at this time. The effect on humidity for water production and energy efficiency for the two types of AWG units are shown in **FIG. 8** and **FIG. 9****,** respectively.

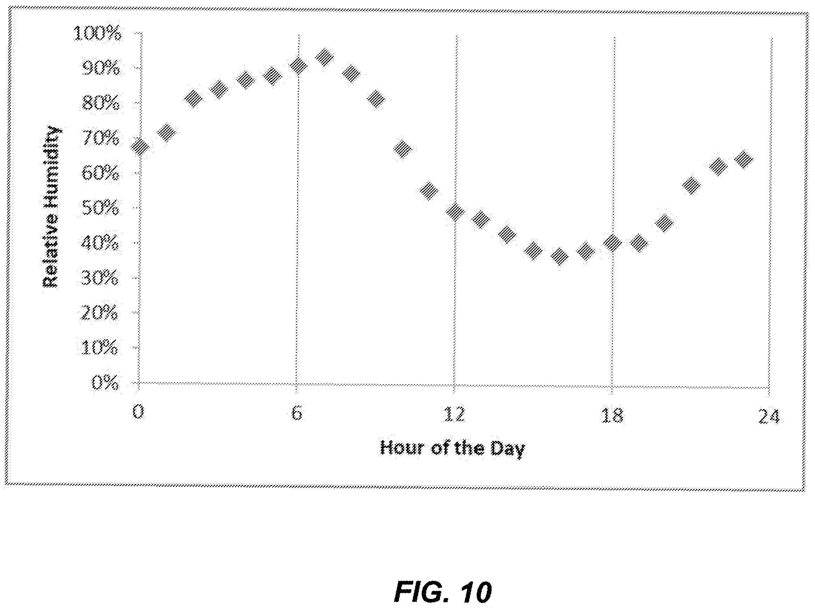

In order to predict the overall water generation and energy efficiency for various combined uses of the two types of AWG units, RH data from central Texas over the course of three days is considered. This data was taken from the National Weather Service during a period of relatively calm weather that is representative for that time of the year. The RH data as a function of hour of the day is shown in **FIG. 10****.** The humidity pattern shown in **FIG. 10** (i.e., the RH drops in the later part of the day) is typical of humidity patterns in most locations. This pattern is consistent with the understanding that warn air holds more moisture, but as no additional moisture is introduced, the relative amount of moisture in the air reaches a maximum and then declines.

Based on this RH data, several usage cases for a large scale AWG platform designed to extract 50,000 gallons of water per day were modeled.
1) A platform using only the large AWG units, running at all times;
2) A platform using only the small AWG units, running at all times;
3) A platform using a 2:1 ratio of large to small AWG units, all units running at all times;
4) A platform using a 2:1 ratio of large to small AWG units, the small AWG units engaged only when the RH is 50% or greater;
5) A platform using a 1:2 ratio of large to small AWG units, all units running at all times; and
6) A platform using a 1:2 ratio of large to small AWG units, the small AWG units engaged only when the RH is 50% or greater.

Using the water generation and power curves, in combination with the average humidity, the performance for each type of AWG unit can be analyzed for each hour of the day in order to determine the overall daily production rates. From this data, the minimum number of AWG units that are needed to meet the daily requirement can be calculated. Similarly, the power that would be required and the net electrical efficiency may also be calculated using this data. The results for each of the cases models are presented in Table 1.

**[TABLE-00001]**

| Case | Water Production (gal/day) | Energy Required (kWh/day) | Electrical Efficiency (kWh/gal) | Number Large Units | ofNumber AWGSmall Units | of AWG |
|---|---|---|---|---|---|---|
| 1 | 51,662 | 80,757 | 1.56 | 33 | 0 | |
| 2 | 52,045 | 47,927 | 0.92 | 0 | 66 | |
| 3 | 50,956 | 73,067 | 1.43 | 26 | 13 | |
| 4 | 53,212 | 74,410 | 1.40 | 28 | 14 | |
| 5 | 50,286 | 62,392 | 1.24 | 16 | 32 | |
| 6 | 52,294 | 59,194 | 1.13 | 18 | 36 | |

Although Case **2,** which uses all small AWG units, is the most energy efficient, it would require 66 small AWG units to meet the daily requirement. In comparison, Case 1 requires 33 large AWG units to meet the daily requirement, which would be a lower investment due to the scaling factor for capital equipment. Although, it was not considered in this analysis, more land space would be required to house 66 small AWG units, which may significantly increase the overall cost.

As can be seen by comparing Case **3** and Case **4,** and Case **5** and Case **6,** there is a benefit in implementing humidity controls for capital costs. For example, although Case **4** requires three additional AWG units in order to meet the daily requirement as compared to Case **3,** there is an improvement in overall energy efficiency in Case **4.** In locations with costly electricity, it may be beneficial to operate as in Case **4,** as the energy savings may offset the additional capital costs.

This difference is even more pronounced in the comparison between Case **5** and. Case **6,** where there is an improvement of about 8.8% in electrical efficiency in Case **6** over Case **5.**

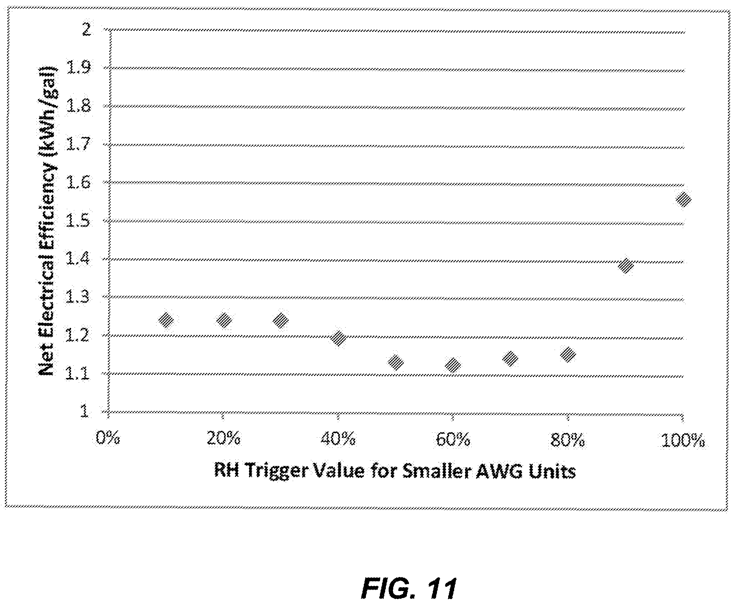

Cases **4** and **6** used a RH trigger value of 50% RH for activating the small AWG units. The RH trigger value can be optimized to improve electrical efficiency within the large scale AWG platform. **FIG. 11** illustrates the overall electrical efficiency changes of the platform as a function of the RH trigger value for Case **6,** while maintaining a minimum water production of 50,000 gal/clay. As shown in **FIG. 11****,** the optimal RH trigger value to obtain the best electrical efficiency for this example is near 50%.

### EXAMPLE 2

A model was created to show the impact of the placement of a large scale AWG platform on regional weather patterns based on forecast models. The model used the existing, open-source Weather Research and Forecasting 3.0 (WRF) program, which was developed over several, years to model and predict the behavior of the atmosphere from the Earth's surface (including the Planetary Boundary Layer) and through the troposphere and higher elevations based on historical and current weather and geographic data, and is maintained by the National. Center for Atmosphere Research. The model was used to simulate the weather patterns over the southern United States, using a 100×100 grid with 10 km spacing. Initial conditions for weather data were taken from the National Weather Service.

The base case represents the NLA and was calculated using weather with initial conditions and forecasting 48 hours in the future, with time increments of 30 seconds. The modified cases, with the large scale AWG platform, included a water moisture consumption term in the Planetary Boundary Layer calculations that accounted for the water recovered by the large scale AWG platform. The amount of water recovered, was a function of the air flow into the large, scale AWG platform, the current moisture content of that air at ground level, a projected water recovery efficiency of 50%, and the total number of AWG units included in the large scale AWG platform.

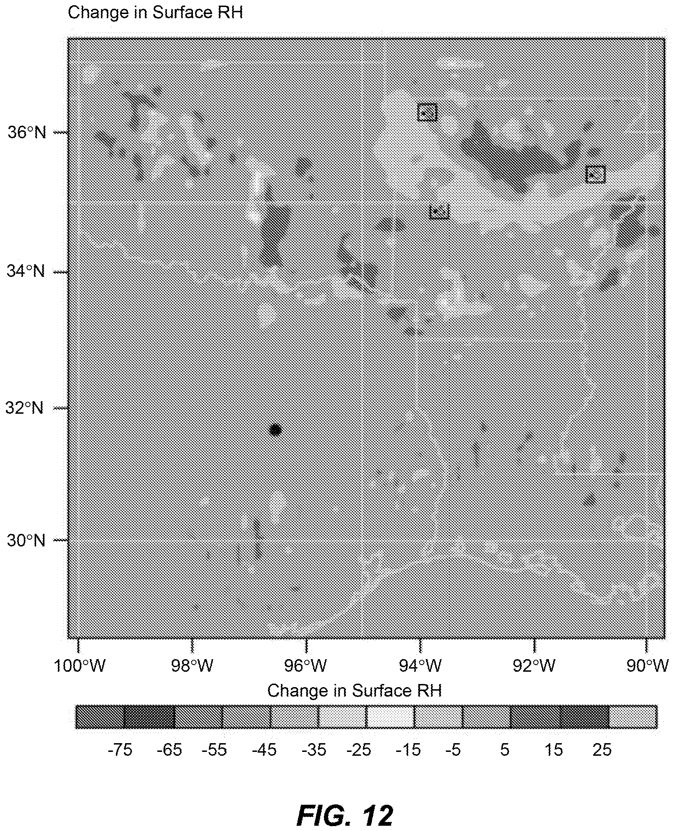

The change in the surface relative humidity (%) between the cases where the large scale AWG platform is operating and the base (NLA) was calculated for cases where the large scale AWG platform contains 100, 500, 1,000, 5,000, and 10,000 AWG units within the large scale AWG platform. In the case of a large scale AWG platform having 100 AWG units (indicated by the black dot), the surface relative humidity differed by plus or minus about 10% in an area that was concentrated northeast of the large scale AWG platform, with some smaller areas of variation were north of the large scale AWG platform, and smaller areas of variation to the east and within closer proximity to the south, as shown in **FIG. 12****.**

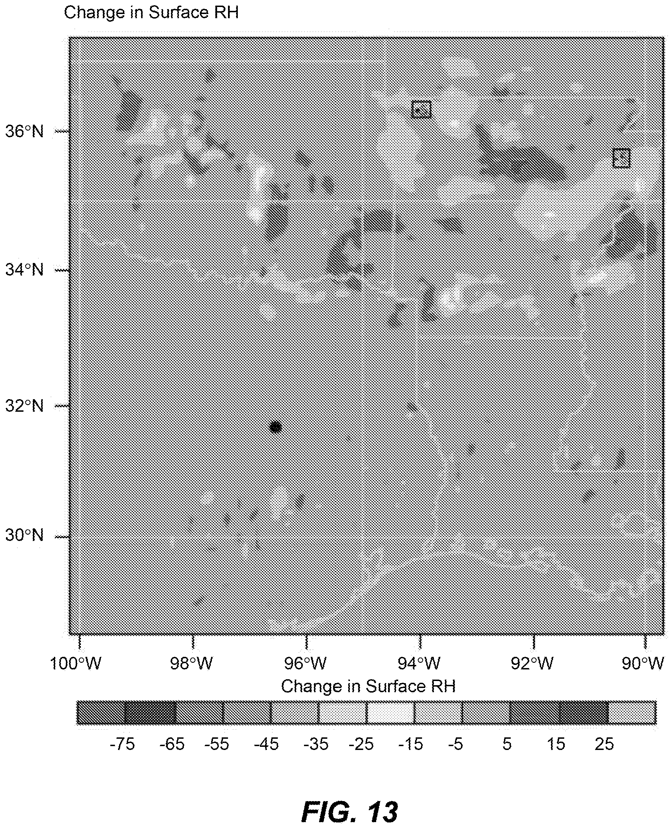

In the case of a large scale AWG platform having 500 AWG units (indicated by the black dot), the surface relative humidity generally differed by, plus or minus about 10% in an area that was concentrated northeast of the large scale AWG platform and some smaller areas of variation north of the large scale AWG platform, as well as small areas where the surface RH was about 20% lower than the location of the large scale AWG platform observed, as shown in **FIG. 13****.** Additionally, smaller areas of variation of plus or minus about 10% surface RH to the east and within closer proximity to the south were also seen. The areas of variation to the east of the large scale AWG platform were smaller, and the areas of variation to the south of the large scale AWG platform were larger than seen with the large scale AWG platform having 100 units.

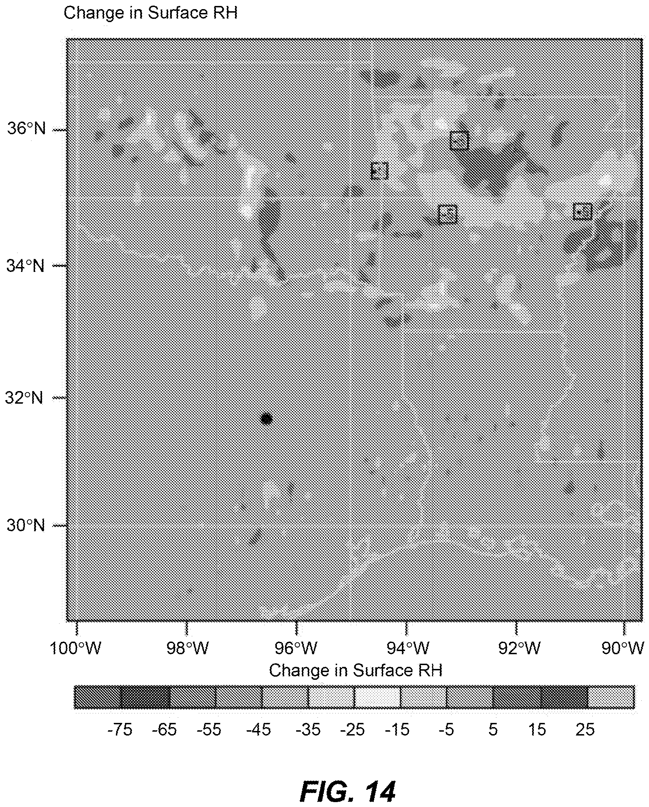

In the case of a large scale AWG platform having 1,000 AWG units (indicated by the black dot), the surface relative humidity generally differed by plus or minus about 10% in an area that was concentrated northeast of the large scale AWG platform, with some variation north of the large scale AWG platform, as well as small areas where the surface RH was about 20% lower than the location of the large scale. AWG platform observed, as shown in **FIG. 14****.** The area, north and northeast of the large, scale AWG platform with lower relative humidity is larger than for the large scale AWG platform having 500 units. However, the smaller areas of variation to the east and within closer proximity to the south appear to be similarly sized.

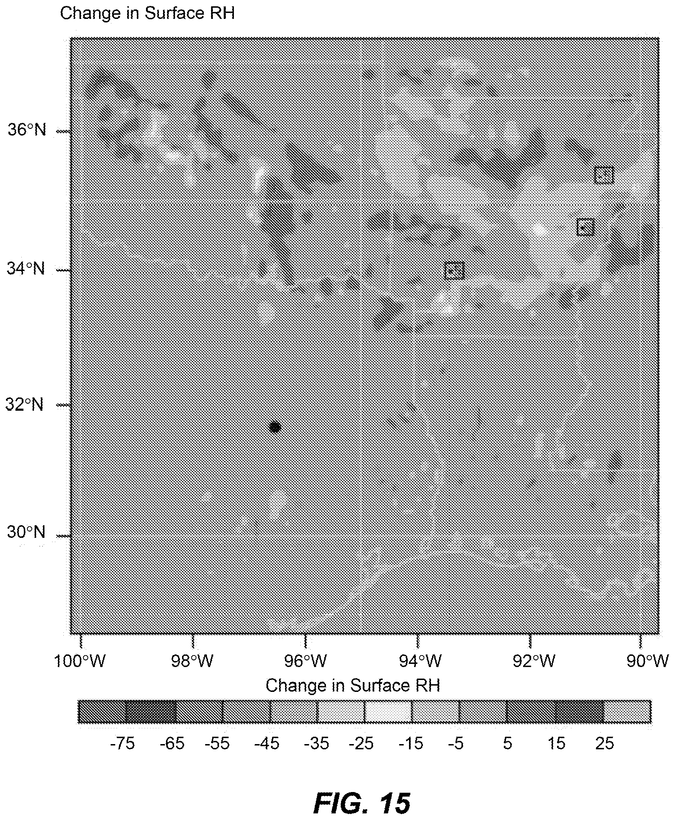

A large scale AWG platform having 5,000 AWG units (indicated by the black dot), has an apparent impact that on the surface RH of a larger area, as shown in **FIG. 15****.** The variation in surface RH was still concentrated northeast of the large scale AWG platform, with some variation north of the large scale AWG platform. The variation is generally plus or minus about 10% of the surface RH of the location of the large scale AWG platform, with some areas having a surface RH that is about 20% lower. The area north and northeast of the large scale AWG platform is larger than for the large scale AWG platform having 1,000 units. However, the smaller areas of variation to the east and within closer proximity to the south appear to remain about the same size.

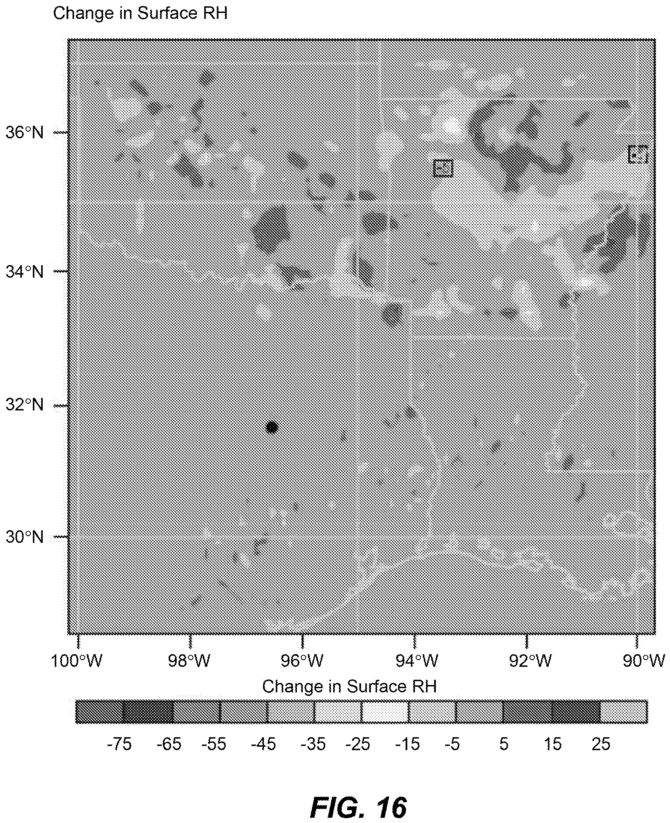

A large scale AWG platform having 10,000 AWG units (indicated by the black dot), appears to impact a similar total area to the large scale AWG platform having 5,000 AWG units, as shown in **FIG. 16****.** The variation in surface RH was still, concentrated northeast of the large scale AWG platform, with some variation north of the large scale AWG platform. The variation is generally plus or minus about 10% of the surface RH of the location of the large scale AWG platform, with some areas having a surface RH that is about 20% lower. The smaller areas of variation to the east and within closer proximity to the south appear to remain about the same size.

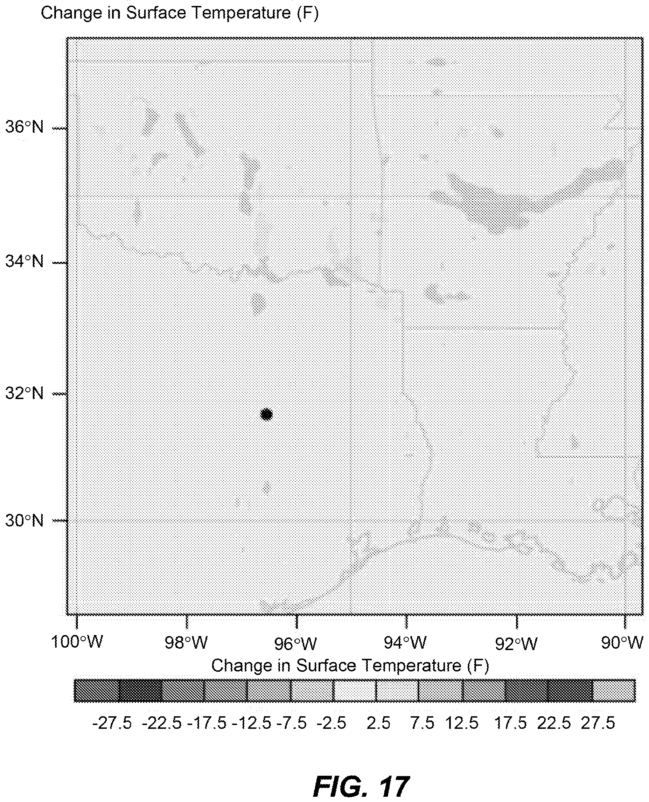

The change in the relative surface temperature in °F. between each case and the base case after 48 hours of forecasting were also modeled. In the case of a large scale AWG platform having 100 AWG units (indicated by the black dot), the surface temperature differed by plus or minus about 5°F. in an area that was concentrated north and northeast of the large scale AWG platform, with some smaller areas of variation to the east and within closer proximity to the south, as shown in **FIG. 17****.**

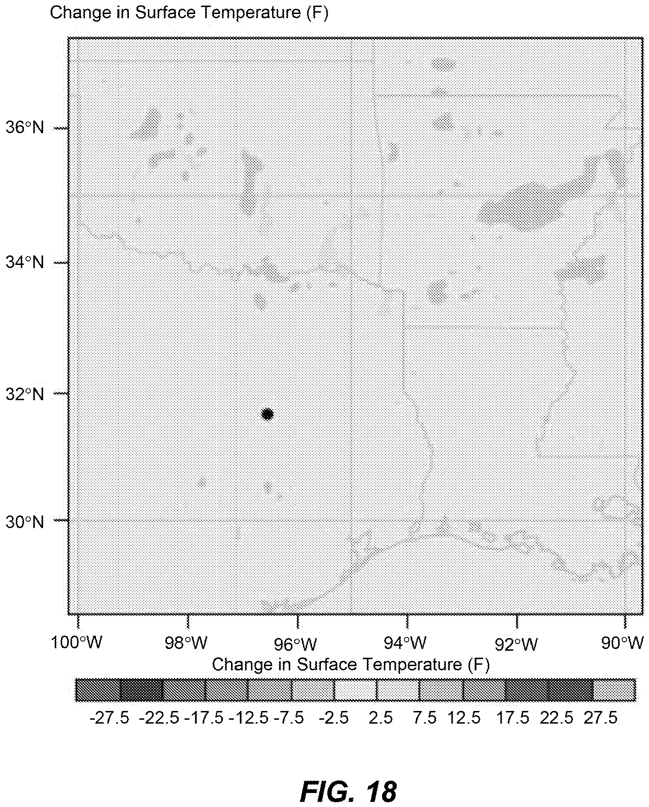

In the case of a large scale AWG platform having 500 AWG units (indicated by the black dot), the surface temperature differed by plus or minus about 5°F. in an area that was concentrated north and northeast of the large scale AWG platform, with some smaller areas of variation to the east and within closer proximity to the south, as shown in **FIG. 18****.** The areas of variation to the north and northeast of the large scale AWG platform were larger than seen with the large scale AWG platform having 100 units.

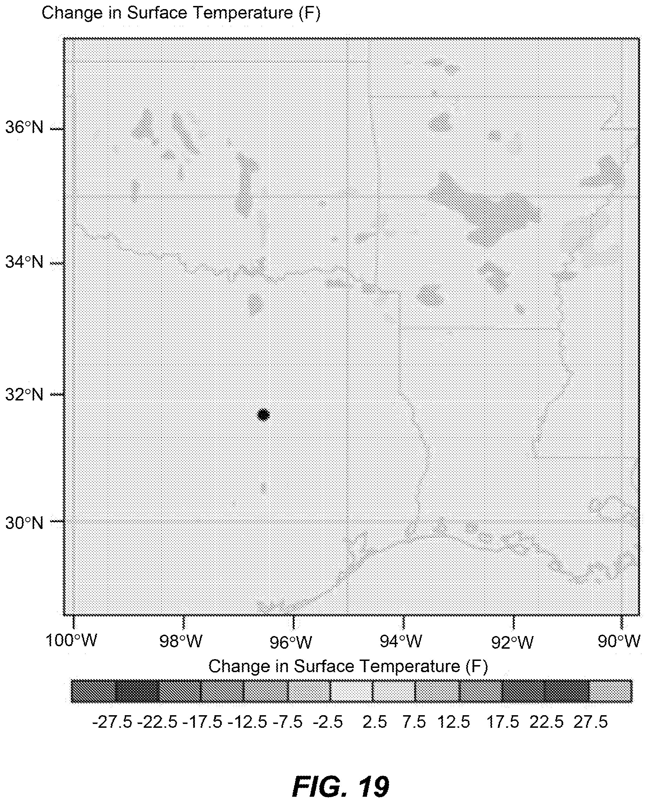

In the case of a large scale AWG platform having 1,000 AWG units (indicated by the black dot), the surface temperature differed by plus or minus about 5° F. in an area that was concentrated north and northeast of the large scale AWG platform, with some smaller areas of variation to the east and within closer proximity to the south, as shown in **FIG. 19****.** The area of variation northeast of the large scale AWG platform is larger than for the large scale AWG platform having 500 units. However, the smaller areas of variation to the east and within closer proximity to the south appear to be similarly sized.

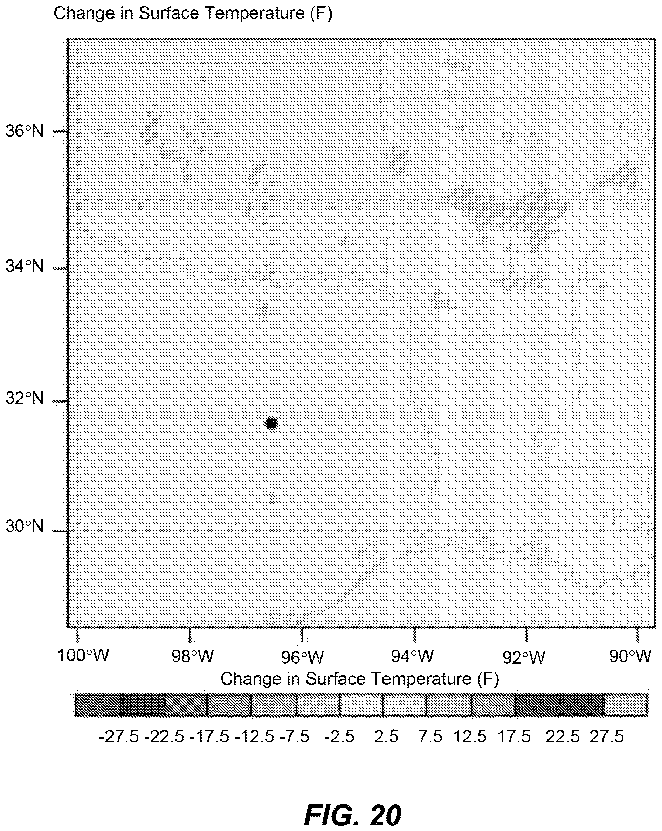

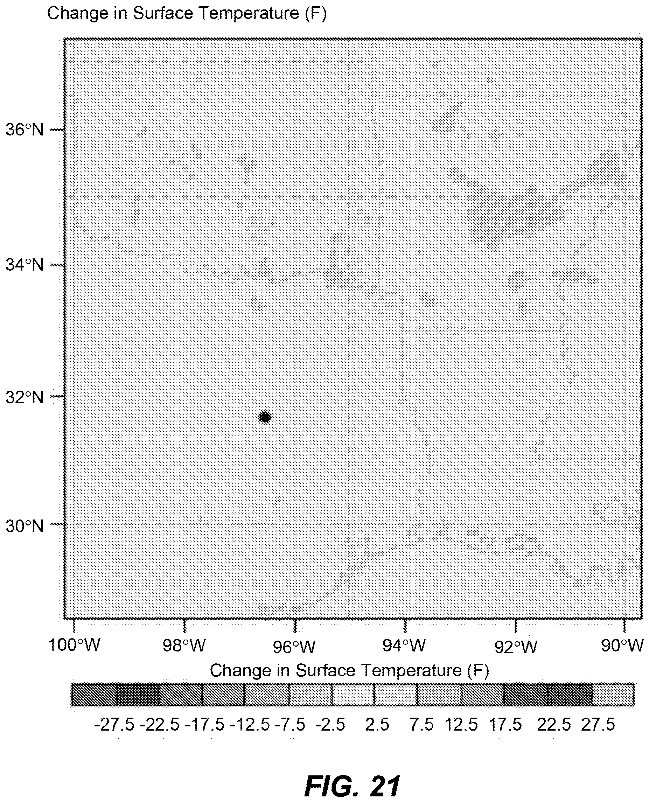

A large scale AWG platform having 5,000 AWG units (indicated by the black dot), has an apparent impact that on the surface temperature of a larger area, as shown in **FIG. 20****.** The variation in surface temperature was concentrated northeast of the large scale AWG platform, with some variation north of the large scale AWG platform. The variation is generally plus or minus about 5° F. of the surface temperature of the location of the large scale AWG platform. The smaller areas of variation to the east and within closer proximity to the south appear to remain similarly sized. A large scale AWG platform having 10,000 AWG units (indicated by the black dot), appears to impact a similar total area to the large scale AWG platform having 5,000 AWG units, as shown in **FIG. 21****.** The variation in surface temperature was still concentrated northeast of the large scale AWG platform, with some variation north of the large scale AWG platform. The variation is generally plus or minus about 5° F. of the surface temperature of the location of the large scale AWG platform. The smaller areas of variation to the east and within closer proximity to the south appear to remain about the same size.

All cases show that the large scale AWG platform affects the weather conditions in areas north and northeast of the large scale AWG platform. Initially these are more focused in the northeast area, but as the size of the large scale AWG platform increases, the northern areas become more affected. While these are not massive changes, with temperature changes no greater than 10°F., and relative humidity changes no greater than 20%, these changes can affect the performance of AWG units within the large scale AWG platform that would be in these areas by a significant amount. AWG units within the large scale AWG platform performance is strongly tied to the relative humidity and temperature. The effects of the large scale AWG platform may be compounded by other natural and man-made phenomenon.

The term "about" has the meaning reasonably ascribed to it by a person of ordinary skill in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

The term "substantially" has the meaning reasonably ascribed to it by a person of ordinary skill in the art when used to describe a physical characteristic of an item, i.e., indicating that the item possesses the referenced characteristic to a significant extent, e.g., to within a range of ±20% of the referenced characteristic; ±19% of the referenced characteristic; ±18% of the referenced characteristic; ±17% of the referenced characteristic; ±16% of the referenced characteristic; ±15% of the referenced characteristic; ±14% of the referenced characteristic; ±13% of the referenced characteristic; ±12% of the referenced characteristic; ±11% of the referenced characteristic; ±10% of the referenced characteristic; ±9% of the referenced characteristic; ±8% of the referenced characteristic; ±7% of the referenced characteristic; ±6% of the referenced characteristic; ±5% of the referenced characteristic; ±4% of the referenced characteristic; ±3% of the referenced characteristic; ±2% of the referenced characteristic; or ±1% of the referenced characteristic. For example, an item may be considered substantially circular if any two measurements of a diameter of the item are within a range of ±20%, ±19%; ±18%; ±17%; ±16%; ±15%; ±14%; ±13%; ±12%; ±11%; ±10%; ±9%; ±8%; ±7%; ±6%; ±5%; ±4%; ±3%; ±2%; or ±1% of each other. When used in conjunction with a comparator, substantially is used to mean that the difference is at least ±20% of the referenced characteristic; +19% of the referenced characteristic; +18% of the referenced characteristic; +17% of the referenced characteristic, +16% of the referenced characteristic; ±15% of the referenced characteristic; ±14% of the referenced characteristic; ±13% of the referenced characteristic; ±12% of the referenced characteristic; +11% of the referenced characteristic; ±10% of the referenced characteristic; ±9% of the referenced characteristic; ±8% of the referenced characteristic; ±7% of the referenced characteristic; ±6% of the referenced characteristic; ±5% of the referenced characteristic; ±4% of the referenced characteristic; ±3% of the referenced characteristic; ±2% of the referenced characteristic; or ±1% of the referenced characteristic.

The terms "a," "an," "the," and similar articles or terms used in the context of describing the disclosure (especially in the context of the following claims) are to be construed to cover both the singular and the plural (i.e., "one or more"), unless otherwise indicated herein or clearly contradicted by context. Ranges of values recited herein are intended to serve as a shorthand method of referring individually to each separate value falling within the range. In the present description, any concentration range, percentage range, ratio range, or integer range is to be understood to include the value of any integer within the recited range and, when appropriate, fractions thereof (such as one tenth and one hundredth of an integer), unless otherwise indicated. Also, any number range recited herein relating to any physical feature, such as size, are to be understood to include any integer within the recited range, unless otherwise indicated. Unless otherwise indicated herein, each individual value is incorporated into the specification as if it were individually recited herein.

The use of the alternative (e.g., "or") should be understood to mean one, both, or any combination thereof of the alternatives. The various embodiments described above can be combined to provide further embodiments. Groupings of alternative elements or embodiments of the disclosure described herein should not be construed as limitations. Each member of a group may be referred to and claimed individually, or in any combination with other members of the group or other elements found herein.

Each embodiment disclosed herein can comprise, consist essentially of, or consist of a particular stated element, step, ingredient, or component. The term "comprise" or "comprises" means "includes, but is not limited to," and allows for the inclusion of unspecified elements, steps, ingredients, or components, even in major amounts. The phrase "consisting of" excludes any element, step, ingredient, or component that is not specified. The phrase "consisting essentially of" limits the scope of the embodiment to the specified elements, steps, ingredients, or components, and to those that do not materially affect the basic and novel characteristics of the claimed disclosure.

The particulars described herein are by way of example and are only for purposes of illustrative discussion of embodiments of the present disclosure. The use of any and all examples, or exemplary language (e.g., "such as") provided herein is merely intended to better illuminate the disclosure and does not pose a limitation on the scope, of the disclosure as claimed. No language in the specification should be construed as indicating any non-claimed element is essential to the practice of the disclosure. Further, all methods described herein can be perfoimed in any suitable order unless otherwise indicated herein or otherwise clearly contradicted by context.

The various embodiments described above can be combined to provide further embodiments embodiments.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

Definitions used in the present disclosure are meant and intended to be controlling in any future construction unless clearly and unambiguously modified in the examples or when application of the meaning renders any construction meaningless or essentially meaningless. In cases where the construction of the term would render it meaningless or essentially meaningless, the definition should be taken from Webster's Dictionary, 3rd Edition or a dictionary known to those of ordinary skill in the art.

Although the subject matter has been described in language specific to structural features or methodological acts, it is to be understood that the subject matter defined in the appended claims is not necessarily limited to the specific features or acts described. Rather, the specific features and acts are disclosed as illustrative forms of implementing the claims.

## Claims

1. A fluid treatment system comprising:
a vessel used to hold fluid, and at least a pair of conductive portions in communication with an interior of the vessel;
a sonic energy generator for applying a sonic signal to at least a portion of a fluid in the vessel;
an electromagnetic field (EMF) generator including at least a pair of contacts coupled to the pair of conductive portions of the vessel, the EMF generator configured to generate an EMF signal that travels through the fluid in the vessel and between the pair of conductive portions of the vessel; and
a controller in communication with said sonic energy generator and said EMF generator that is configured to synchronize said EMF signal and said sonic signal according to one of the group of:
(1) wherein the sonic signal has a frequency, and the EMF signal is pulsed regularly at a frequency that is an integer value of a harmonic of the frequency of the sonic signal;
(2) wherein the sonic signal has a frequency, and the EMF signal also has a frequency, and wherein the frequency of one of said signals is n times the frequency of the other of said signals, where n is an integer other than zero;
(3) wherein the EMF signal has a waveform and the sonic signal is a harmonic of the waveform of the EMF signal;
(4) wherein the sonic signal has a frequency, and the EMF signal is pulsed regularly at the same frequency as the sonic signal with no phase shift;
(5) wherein the sonic signal has a frequency, and the EMF signal is pulsed at the same frequency as the sonic signal such that the EMF signal occurs at an onset of a compression wave of the sonic signal, and
(6) combinations thereof.

2. The system of claim 1 wherein the controller is selected from the group of: a batch process, a continuous process, a standalone control system, part of larger control system, a sensor network and combinations thereof, for the production, storage, and distribution of at least one of water, power, data and combinations thereof.

3. The system of claim 1 wherein the system operates as a trading desk for one of: water trading, power trading, data commodities trading and combinations thereof.

4. The system of claim 1 wherein the controller is enabled for wired or wireless internet capabilities and is in communication with an IoT system that tracks weather patterns at a large scale.

5. The system of claim 4 wherein the controller uses data provided over the internet to model predicted weather conditions.

6. The system of claim 1 wherein the system operates as a node within a nodal network of one of water, power, data generation, storage, and distribution; and includes data sharing throughout the nodal network to achieve optimum intra-platform and inter-platform performance for the entire nodal network.

7. The system of claim 6 wherein the nodal network is capable of fault tolerant operations, addressing redundancy, resiliency, and stability of system operations during nominal, optimal, and emergency use.

8. The system of claim 6, where the system responds to information communicated from the nodal network using one of: real-time model analysis, predictive modeling and control, neural networks, fuzzy logic systems, genetic algorithms, expert systems, software agents, knowledge management (KM) systems for data mining, cloud computing, parallel computing, distributed computing through wired or wireless communication methods utilized in Internet of Things (IoT), and combinations thereof.

9. The system of claim 6, where the nodal network is a combination of a physically wired and wireless systems and uses security and encryption technologies and methodologies utilized in a field selected from: buildings management IoT (BloT), smart cities, infrastructure, utilities, healthcare systems, insurance industry, manufacturing, and combinations thereof.

10. The system of claim 1, further comprising at least one atmospheric water generator (AWG) unit for providing water to said vessel.

11. The system of claim 10 wherein the production rate of the at least one AWG unit is highest when operating in a natural local atmosphere (NLA).

12. The system of claim 10 wherein the at least one AWG unit is part of a large-scale AWG platform, and the at least one AWG unit is arranged to maximize the moisture content of the ambient air and production rate for a given location and AWG unit efficiency based on local weather patterns.

13. The system of claim 10 wherein the at least one atmospheric water generator (AWG) unit comprises more than one type of AWG unit, each type having a different efficiency based on relative humidity; wherein one or more AWG units of one type are activated based on relative humidity, maximization of energy efficiency, maximization of water recovery, and combinations thereof.

14. A method comprising:
receiving a fluid in a vessel; wherein the vessel includes at least a pair of conductive portions in communication with an interior of the vessel;
applying a sonic signal to at least a portion of the fluid;
applying an electromagnetic field (EMF) signal that travels through the fluid in the vessel and between the pair of conductive portions of the vessel; and
synchronizing said sonic signal and said EMF signal according to a member selected from the group of:
(1) wherein said sonic signal has a frequency, and said EMF signal is pulsed regularly at a frequency that is an integer value of a harmonic of the frequency of the sonic signal;
(2) wherein said sonic signal has a frequency and said EMF signal has a frequency, and said signals are applied such that the frequency of one of said signals is n times the frequency of the other of said signals, where n is an integer other than zero;
(3) wherein said sonic signal has a waveform and said EMF signal has a waveform, and said signals are applied such that said sonic signal is a harmonic of the waveform of the EMF signal;
(4) wherein said sonic signal has a frequency and said EMF signal has a frequency, and said EMF signal is pulsed regularly at the same frequency as the sonic signal with no phase shift;
(5) wherein said sonic signal has a frequency and said EMF signal has a frequency, and the application of said EMF signal and said sonic signal is timed such that the EMF signal occurs at an onset of a compression wave of the sonic signal; and
(6) combinations thereof.

15. The method of claim 14 further comprising the steps of:
activating at least one of a plurality of atmospheric water generator (AWG) units;
extracting water from ambient air by at least one of the plurality of AWG units;
receiving said water into the vessel; and
deactivating at least one of said plurality of AWG units based on data regarding at least one of: geography, climate, weather, water, power, and combinations thereof; wherein the data is received in real-time, produced by predictive modeling, and combinations thereof.
